(19) Europäisches Patentamt
European Patent Office
Office européen des brevets

(11) **EP 2 934 413 B1**

(12) **EUROPEAN PATENT SPECIFICATION**

(45) Date of publication and mention
of the grant of the patent:
**05.07.2017 Bulletin 2017/27**

(51) Int Cl.:
*A61F 13/532* (2006.01)    *A61F 13/539* (2006.01)
*A61F 13/15* (2006.01)

(21) Application number: **13820772.5**

(86) International application number:
**PCT/EP2013/077867**

(22) Date of filing: **20.12.2013**

(87) International publication number:
**WO 2014/096439 (26.06.2014 Gazette 2014/26)**

(54) **FLUID-ABSORBENT ARTICLE**

FLUID-ABSORBIERENDEN ARTIKEL

ARTICLE ABSORBANT DES FLUIDES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2012 EP 12199281
21.12.2012 US 201261740752 P**

(43) Date of publication of application:
**28.10.2015 Bulletin 2015/44**

(73) Proprietors:
• **Bostik, Inc.**
**Wauwatosa, Wisconsin 53226 (US)**
• **BASF SE**
**67056 Ludwigshafen am Rhein (DE)**

(72) Inventors:
• **BAUDUIN, Christophe**
**68723 Planckstadt (DE)**
• **CHARTREL, Jean François**
**F-60400 Cuts (FR)**
• **DANIEL, Thomas**
**67165 Waldsee (DE)**
• **LIGER, Julien**
**F-60280 Margny Les Compiègnes (FR)**

(74) Representative: **Hubert, Philippe et al
Cabinet Beau de Loménie
158, rue de l'Université
75340 Paris Cedex 07 (FR)**

(56) References cited:
EP-A1- 1 621 166       WO-A1-2012/048878
WO-A2-2010/133529     US-A1- 2002 095 127

**Description**

RELATED APPLICATIONS

This application claims the benefit of the European application EP12199281.2 and U.S. Prov. 61/740,752 filed Dec 21, 2012.

DESCRIPTION

The present invention relates to absorbent cores especially ultrathin fluid absorbent cores comprising fluid-absorbent polymers in a plurality of pockets, wherein the particle size distribution of the fluid-absorbent polymers in the pockets vary by not more than 15% between the pockets.

The production of fluid-absorbent articles is described in the monograph "Modern Superabsorbent Polymer Technology", F.L. Buchholz and A.T. Graham, Wiley-VCH, 1998, pages 252 to 258.

Fluid-absorbent articles such as disposable diapers typically comprise an upper liquid-pervious layer, a lower liquid-impervious layer, and a fluid-absorbent core between the upper and the lower layer. The fluid-absorbent cores typically comprise fluid-absorbent polymers and fibers.

Ultrathin fluid-absorbent cores can be formed by immobilization of fluid-absorbent polymer particles on a nonwoven by using hotmelt adhesives, i.e. forming longitudinal strips or discrete spots and/or by joining two layers of e.g. nonwoven partially together forming a plurality of pockets immobilizing the fluid-absorbent particles.

The preparation of ultrathin fluid-absorbent cores is described, for example, in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, WO 2008/155722 A2 and WO 2012/052172.

In fluid-absorbent cores comprising at least 80 wt.% of fluid-absorbent polymer particles the size distribution of the fluid-absorbent polymer is very important for the performance of the fluid absorbent article. As large fluid absorbent particles swell very slowly and therefore decrease the rate of fluid absorption and e.g. small particles (Fines) swell rapidly but decrease the permeability of the core which may lead to "gel blocking".

In order to reduce or eliminate these effects the particle size distribution of the fluid-absorbent particles needs to be adjusted.

For example WO 98/37149 discloses fluid-absorbent particles having a mass median particle size equal to or greater than about 400 $\mu$m, which is mixed with hydrophilic fibrous material.

Whereas US 2005/0209352 describes fluid-absorbent particles
having a mass average particle diameter (D50) from 200 to 600 $\mu$m and
whereas 95 to 100 wt % having a particle diameter from less than 850 $\mu$m to not less than 150 $\mu$m with respect to 100 wt % of whole particulate fluid-absorbing agent, and
having a logarithmic standard deviation ($\sigma\zeta$) of the particle size distribution from 0.25 to 0.45.

EP 1 730 218 discloses fluid-absorbent particles having a mass median particle size (D50) of 200 to 400$\mu$m and a ratio of particles smaller than 600 $\mu$m and not smaller than 150$\mu$m to be 95 to 100% by weight.

But there may be further parameters which influence the performance of fluid-absorbent articles, especially of absorbent cores.

Therefore it was an object of the present invention to further improve the properties of fluid-absorbent cores, i. e. the absorption properties.

The object is achieved by fluid-absorbent cores comprising an upper layer (A), a lower layer (B), at least 80 wt% fluid-absorbent polymer particles (C) between (A) and (B), the upper layer and the lower layer being at least partially joint together by attachments forming a sandwich-like structure with the unattached regions between the upper layer and the lower layer forming pockets containing fluid-absorbent polymer particles, wherein the particle size distribution (PSD) of the fluid-absorbent polymer particles in one pocket varies from the PSD of the fluid-absorbent polymer particles in any other pocket by not more than 15 %.

In a preferred embodiment of the fluid-absorbent core the PSD of the fluid-absorbent polymer particles in every pocket varies from the PSD of the fluid-absorbent polymer particles in any other pocket by not more than 10 %, preferably by not more than 5 %, more preferably by not more than 2%.

Furthermore it may be that the PSD of the fluid-absorbent polymer particles in every pocket is the same.

The fluid-absorbent core comprises at least 80% by weight, preferably at least 85% by weight more preferably at least 90% by weight most preferably at least 100% by weight of fluid-absorbent polymer particles

The fluid absorbent core according to the invention comprises not more than 20% by weight, preferably not more than 15% by weight, more preferably 10% by weight most preferably 0% by weight of the total of cellulose and/or synthetic non-cellulose based fibers.

In a preferred embodiment of the present invention the fluid absorbent core is essentially free of cellulose based fibres (fluffless fluid-absorbent core).

DETAILED DESCRIPTION OF THE INVENTION

A. Definitions

As used herein, the term "fluid-absorbent composition" refers to a component of the fluid-absorbent article which is primarily responsible for the fluid handling of the fluid-absorbent article including acquisition, transport, distribution and storage of body fluids.

As used herein, the term "absorbent core" refers to a fluid-absorbent composition of 80-100 wt% fluid-absorbent polymer particles and 0-20 wt% of a fibrous material. The fluid-absorbent core is primarily responsible for the fluid handling of the fluid-absorbent article including acquisition, transport, distribution and storage of body fluids.

As used herein, the term "layer" refers to a fluid-absorbent composition whose primary dimension is along its length and width. It should be known that the term "layer" is not necessarily limited to single layers or sheets of the fluid-absorbent composition. Thus a layer can comprise laminates, composites, combinations of several sheets or webs of different materials.

As used herein, the term "x-dimension" refers to the length, and the term "y-dimension" refers to the width of the fluid-absorbent composition, layer, core or article. Generally, the term "x-y dimension" refers to the plane, orthogonal to the height or thickness of the fluid-absorbent composition, layer, core or article.

As used herein, the term "z-dimension" refers to the dimension orthogonal to the length and width of the fluid-absorbent composition, layer, core or article. Generally, the term "z-dimension" refers to the height of the fluid-absorbent composition.

As used herein, the term "chassis" refers to fluid-absorbent material comprising the upper liquid-pervious layer and the lower liquid-impervious layer.

As used herein, the term "basis weight" indicates the weight of the fluid-absorbent core per square meter and it includes the chassis of the fluid-absorbent article. The basis weight is determined at discrete regions of the fluid-absorbent core: the front overall average is the basis weight of the fluid-absorbent core 5.5 cm forward of the center of the core to the front distal edge of the core; the insult zone is the basis weight of the fluid-absorbent core 5.5 cm forward and 0.5cm backwards of the center of the core; the back overall average is the basis weight of the fluid-absorbent core 0.5 cm backward of the center of the core to the rear distal edge of the core.

As used herein, the term "density" indicates the weight of the fluid-absorbent core per volume and it includes the chassis of the fluid-absorbent article. The density is determined at discrete regions of the fluid-absorbent core: the front overall average is the density of the fluid-absorbent core 5.5 cm forward of the center of the core to the front distal edge of the core; the insult zone is the density of the fluid-absorbent core 5.5 cm forward and 0.5cm backwards of the center of the core; the back overall average is the density of the fluid-absorbent core 0.5 cm backward of the center of the core to the rear distal edge of the core.

Further, it should be understood, that the term "upper" refers to fluid-absorbent compositions which are nearer to the wearer of the fluid-absorbent article. Generally, the topsheet is the nearest composition to the wearer of the fluid-absorbent article, hereinafter described as "upper liquid-pervious layer". Contrarily, the term "lower" refers to fluid-absorbent compositions which are away from the wearer of the fluid-absorbent article. Generally, the backsheet is the composition which is furthermost away from the wearer of the fluid-absorbent article, hereinafter described as "lower liquid-impervious layer".

As used herein, the term "liquid-pervious" refers to a substrate or a layer thus permitting liquids, i.e. body fluids such as urine, menses and/or vaginal fluids to readily penetrate through its thickness.

As used herein, the term "liquid-impervious" refers to a substrate or a layer that does not allow body fluids to pass through in a direction generally perpendicular to the plane of the layer at the point of liquid contact under ordinary use conditions.

As used herein, the term "hydrophilic" refers to the wettability of fibers by water deposited on these fibers. The term "hydrophilic" is defined by the contact angle and surface tension of the body fluids. According to the definition of Robert F. Gould in the 1964 American Chemical Society publication "Contact angle, wettability and adhesion", a fiber is referred to as hydrophilic, when the contact angle between the liquid and the fiber, especially the fiber surface, is less than 90° or when the liquid tends to spread spontaneously on the same surface.

Contrarily, term "hydrophobic" refers to fibers showing a contact angle of greater than 90° or no spontaneously spreading of the liquid across the surface of the fiber.

As used herein, the term "section" or "zone" refers to a definite region of the fluid-absorbent composition.

As used herein, the term "article" refers to any three-dimensional solid material being able to acquire and store fluids discharged from the body. Preferred articles according to the present invention are disposable fluid-absorbent articles that are designed to be worn in contact with the body of a user such as disposable fluid-absorbent pantiliners, sanitary napkins, catamenials, incontinence inserts/pads, diapers, training pant diapers, breast pads, interlabial inserts/pads and the like.

As used herein, the term "body fluids" refers to any fluid produced and discharged by human or animal body, such as urine, menstrual fluids, faeces, vaginal secretions and the like.

B. Fluid-absorbent polymer Particles

The production of fluid-absorbing polymer particles is described in the monograph "Modern Superabsorbent Polymer Technology", F. L. Buchholz and A. T. Graham, Wiley-VCH, 1998, pages 71 to 103.

The fluid-absorbing polymer particles are produced, for example, by polymerizing a monomer solution or suspension comprising

a) at least one ethylenically unsaturated monomer which bears acid groups and may be at least partly neutralized,
b) at least one crosslinker,
c) at least one initiator,
d) optionally one or more ethylenically unsaturated monomers copolymerizable with the monomers mentioned under a) and
e) optionally one or more water-soluble polymers,

and are typically water-insoluble.

The fluid-absorbent polymer particles are typically insoluble but swellable in water.

The monomers a) are preferably water-soluble, i.e. the solubility in water at 23°C is typically at least 1 g/100 g of water, preferably at least 5 g/100 g of water, more preferably at least 25 g/100 g of water, most preferably at least 35 g/100 g of water.

Suitable monomers a) are, for example, ethylenically unsaturated carboxylic acids such as acrylic acid, methacrylic acid, maleic acid, and itaconic acid. Particularly preferred monomers are acrylic acid and methacrylic acid. Very particular preference is given to acrylic acid.

Further suitable monomers a) are, for example, ethylenically unsaturated sulfonic acids such as vinylsulfonic acid, styrenesulfonic acid and 2-acrylamido-2-methylpropanesulfonic acid (AMPS).

Impurities may have a strong impact on the polymerization. Preference is given to especially purified monomers a). Useful purification methods are disclosed in WO 2002/055469 A1, WO 2003/078378 A1 and WO 2004/035514 A1. A suitable monomer a) is according to WO 2004/035514 A1 purified acrylic acid having 99.8460% by weight of acrylic acid, 0.0950% by weight of acetic acid, 0.0332% by weight of water, 0.0203 by weight of propionic acid, 0.0001% by weight of furfurals, 0.0001% by weight of maleic anhydride, 0.0003% by weight of diacrylic acid and 0.0050% by weight of hydroquinone monomethyl ether.

Polymerized diacrylic acid is a source for residual monomers due to thermal decomposition. If the temperatures during the process are low, the concentration of diacrylic acid is no more critical and acrylic acids having higher concentrations of diacrylic acid, i.e. 500 to 10,000 ppm, can be used for the inventive process.

The content of acrylic acid and/or salts thereof in the total amount of monomers a) is preferably at least 50 mol%, more preferably at least 90 mol%, most preferably at least 95 mol%.

Optionally, it is possible to add to the monomer solution, or to starting materials thereof, one or more chelating agents for masking metal ions, for example iron, for the purpose of stabilization. Suitable chelating agents are, for example, alkali metal citrates, citric acid, alkali metal tartrates alkali metal lactates and glycolates, pentasodium triphosphate, ethylenediamine tetraacetate, nitrilotriacetic acid, and all chelating agents known under the Trilon® name, for example Trilon® C (pentasodium diethylenetriaminepentaacetate), Trilon® D (trisodium (hydroxyethyl)-ethylenediaminetriacetate), and Trilon® M (methylglycinediacetic acid).

The monomers a) comprise typically polymerization inhibitors, preferably hydroquinone monoethers, as inhibitor for storage.

The monomer solution comprises preferably up to 250 ppm by weight, more preferably not more than 130 ppm by weight, most preferably not more than 70 ppm by weight, preferably not less than 10 ppm by weight, more preferably not less than 30 ppm by weight and especially about 50 ppm by weight of hydroquinone monoether, based in each case on acrylic acid, with acrylic acid salts being counted as acrylic acid. For example, the monomer solution can be prepared using acrylic acid having appropriate hydroquinone monoether content. The hydroquinone monoethers may, however, also be removed from the monomer solution by absorption, for example on activated carbon.

Preferred hydroquinone monoethers are hydroquinone monomethyl ether (MEHQ) and/or alpha-tocopherol (vitamin E).

Suitable crosslinkers b) are compounds having at least two groups suitable for cross-linking. Such groups are, for example, ethylenically unsaturated groups which can be polymerized by a free-radical mechanism into the polymer chain and functional groups which can form covalent bonds with the acid groups of monomer a). In addition, polyvalent metal ions which can form coordinate bond with at least two acid groups of monomer a) are also suitable crosslinkers b).

The crosslinkers b) are preferably compounds having at least two free-radically polymerizable groups which can be polymerized by a free-radical mechanism into the polymer network. Suitable crosslinkers b) are, for example, ethylene glycol dimethacrylate, diethylene glycol diacrylate, polyethylene glycol diacrylate, allyl methacrylate, trimethylolpropane triacrylate, triallylamine, tetraallylammonium chloride, tetraallyloxye-thane, as described in EP 0 530 438 A1, di- and triacrylates, as described in EP 0 547 847 A1, EP 0 559 476 A1, EP 0 632 068 A1, WO 93/21237 A1, WO 2003/104299 A1, WO 2003/104300 A1, WO 2003/104301 A1 and in DE 103 31 450 A1, mixed acrylates which, as well as acrylate groups, comprise further ethylenically unsaturated groups, as described in DE 103 314 56 A1 and DE 103 55 401 A1, or crosslinker mixtures, as described, for example, in DE 195 43 368 A1, DE 196 46 484 A1, WO 90/15830 A1 and WO 2002/32962 A2.

Suitable crosslinkers b) are in particular pentaerythritol triallyl ether, tetraallyloxy-ethane, N,N'-methylenebisacrylamide, 15-tuply ethoxylated trimethylolpropane, polyethylene glycol diacrylate, trimethylolpropane triacrylate and triallylamine.

Very particularly preferred crosslinkers b) are the polyethoxylated and/or - propoxylated glycerols which have been esterified with acrylic acid or methacrylic acid to give di- or triacrylates, as described, for example in WO 2003/104301 A1. Di- and/or triacrylates of 3- to 10-tuply ethoxylated glycerol are particularly advantageous. Very particular preference is given to di- or triacrylates of 1- to 5-tuply ethoxylated and/or propoxylated glycerol. Most preferred are the triacrylates of 3- to 5-tuply ethoxylated and/or propoxylated glycerol and especially the triacrylate of 3-tuply ethoxylated glycerol.

The amount of crosslinker b) is preferably from 0.05 to 1.5% by weight, more preferably from 0.1 to 1% by weight, most preferably from 0.3 to 0.6% by weight, based in each case on monomer a). On increasing the amount of crosslinker b) the centrifuge retention capacity (CRC) decreases and the absorption under a pressure of 21.0 g/cm$^2$ (AUL) passes through a maximum.

The initiators c) used may be all compounds which disintegrate into free radicals under the polymerization conditions, for example peroxides, hydroperoxides, hydrogen peroxide, persulfates, azo compounds and redox initiators. Preference is given to the use of water-soluble initiators. In some cases, it is advantageous to use mixtures of various initiators, for example mixtures of hydrogen peroxide and sodium or potassium peroxo-disulfate. Mixtures of hydrogen peroxide and sodium peroxodisulfate can be used in any proportion.

The initiators are used in customary amounts, for example in amounts of from 0.001 to 5% by weight, preferably from 0.01 to 2% by weight, based on the monomers a).

Particularly preferred initiators c) are azo initiators such as 2,2'-azobis[2-(2-imidazolin-2-yl)propane] dihydrochloride and 2,2'-azobis[2-(5-methyl-2-imidazolin-2-yl)propane] dihydrochloride, and photoinitiators such as 2-hydroxy-2-methylpropiophenone and 1-[4-(2-hydroxyethoxy)phenyl]-2-hydroxy-2-methyl-1-propan-1-one, redox initiators such as sodium persulfate/hydroxymethylsulfinic acid, ammonium peroxodisulfate/hydroxymethylsulfinic acid, hydrogen peroxide/hydroxymethylsulfinic acid, sodium persulfate/ascorbic acid, ammonium peroxodisulfate/ascorbic acid and hydrogen peroxide/ascorbic acid, photoinitiators such as 1-[4-(2-hydroxyethoxy)phenyl]-2-hydroxy-2-methyl-1-propan-1-one, and mixtures thereof. The reducing component used is, however, preferably a mixture of the sodium salt of 2-hydroxy-2-sulfinatoacetic acid, the disodium salt of 2-hydroxy-2-sulfonatoacetic acid and sodium bisulfite. Such mixtures are obtainable as Brüggolite® FF6 and Brüggolite® FF7 (Brüggemann Chemicals; Heilbronn; Germany).

Ethylenically unsaturated monomers d) copolymerizable with the ethylenically unsaturated monomers a) are, for example, acrylamide, methacrylamide, hydroxyethyl acrylate, hydroxyethyl methacrylate, dimethylaminoethyl methacrylate, dimethylaminoethyl acrylate, dimethylaminopropyl acrylate, diethylaminopropyl acrylate, dimethylaminoethyl methacrylate, diethylaminoethyl methacrylate.

Useful water-soluble polymers e) include polyvinyl alcohol, polyvinylpyrrolidone, starch, starch derivatives, modified cellulose, such as methylcellulose or hydroxyethylcellulose, gelatin, polyglycols or polyacrylic acids, polyesters and polyamides, polylactic acid, polyvinylamine, preferably starch, starch derivatives and modified cellulose.

The water content of the monomer solution is preferably from 40 to 75% by weight, more preferably from 45 to 70% by weight, most preferably from 50 to 65% by weight. It is also possible to use monomer suspensions, i.e. monomer solutions with excess monomer a), for example sodium acrylate. With rising water content, the energy requirement in the subsequent drying rises, and, with falling water content, the heat of polymerization can only be removed inadequately.

For optimal action, the preferred polymerization inhibitors require dissolved oxygen. The monomer solution can therefore be freed of dissolved oxygen before the polymerization by inertization, i.e. flowing an inert gas through, preferably nitrogen or carbon dioxide. The oxygen content of the monomer solution is preferably lowered before the polymerization to less than 1 ppm by weight, more preferably to less than 0.5 ppm by weight, most preferably to less than 0.1 ppm by weight.

Suitable reactors are, for example, kneading reactors or belt reactors. In the kneader, the polymer gel formed in the polymerization of an aqueous monomer solution or suspension is comminuted continuously by, for example, contrarotatory stirrer shafts, as described in WO 2001/038402 A1. Polymerization on a belt is described, for example, in DE 38 25 366 A1 and US 6,241,928. Polymerization in a belt reactor forms a polymer gel, which has to be comminuted in a further process step, for example in an extruder or kneader.

However, it is also possible to dropletize an aqueous monomer solution and to polymerize the droplets obtained in a heated carrier gas stream. This allows the process steps of polymerization and drying to be combined, as described in WO 2008/040715 A2 and WO 2008/052971 A1.

The acid groups of the resulting polymer gels have typically been partially neutralized. Neutralization is preferably carried out at the monomer stage. This is typically done by mixing in the neutralizing agent as an aqueous solution or preferably also as a solid. The degree of neutralization is preferably from 25 to 85 mol%, for "acidic" polymer gels more preferably from 30 to 60 mol%, most preferably from 35 to 55 mol%, and for "neutral" polymer gels more preferably from 65 to 80 mol%, most preferably from 70 to 75 mol%, for which the customary neutralizing agents can be used, preferably alkali metal hydroxides, alkali metal oxides, alkali metal carbonates or alkali metal hydrogencarbonates and also mixtures thereof. Instead of alkali metal salts, it is also possible to use ammonium salts, such as the salt of triethanolamine. Particularly preferred alkali metals are sodium and potassium, but very particular preference is given to sodium hydroxide, sodium carbonate or sodium hydrogencarbonate and also mixtures thereof.

However, it is also possible to carry out neutralization after the polymerization, at the stage of the polymer gel formed in the polymerization. It is also possible to neutralize up to 40 mol%, preferably 10 to 30 mol% and more preferably 15 to 25 mol% of the acid groups before the polymerization by adding a portion of the neutralizing agent actually to the monomer solution and setting the desired final degree of neutralization only after the polymerization, at the polymer gel stage. When the polymer gel is neutralized at least partly after the polymerization, the polymer gel is preferably comminuted mechanically, for example by means of an extruder, in which case the neutralizing agent can be sprayed, sprinkled or poured on and then carefully mixed in. To this end, the gel mass obtained can be repeatedly extruded for homogenization.

The polymer gel is then preferably dried with a belt drier until the residual moisture content is preferably 0.5 to 15% by weight, more preferably 1 to 10% by weight, most preferably 2 to 8% by weight, the residual moisture content being determined by EDANA recommended test method No. WSP 230.2-05 "Moisture Content". In the case of too high a residual moisture content, the dried polymer gel has too low a glass transition temperature Tg and can be processed further only with difficulty. In the case of too low a residual moisture content, the dried polymer gel is too brittle and, in the subsequent comminution steps, undesirably large amounts of polymer particles with an excessively low particle size (fines) are obtained. The solids content of the gel before the drying is preferably from 25 to 90% by weight, more preferably from 35 to 70% by weight, most preferably from 40 to 60% by weight. Optionally, it is, however, also possible to use a fluidized bed drier or a paddle drier for the drying operation.

Thereafter, the dried polymer gel is ground and classified, and the apparatus used for grinding may typically be single- or multistage roll mills, preferably two- or three-stage roll mills, pin mills, hammer mills or vibratory mills.

The mean particle size of the polymer particles removed as the product fraction is preferably at least 200 $\mu$m, more preferably from 250 to 600 $\mu$m, very particularly from 300 to 500 $\mu$m. The mean particle size of the product fraction may be determined by means of EDANA recommended test method No. WSP 220.3.10 "Particle Size Distribution", where the proportions by mass of the screen fractions are plotted in cumulative form and the mean particle size is determined graphically. The mean particle size here is the value of the mesh size which gives rise to a cumulative 50% by weight.

Preferably the Logarithmic standard deviation ($\sigma\zeta$) of the particle size distribution is narrow. This means a small value of $\sigma\zeta$.

According to EP 1 730 218 the logarithmic standard deviation may be determined as:

At first the particle size distribution is determined by sieving. The oversize percentages R at each particle size were plotted on a logarithmic scale. Logarithmic standard deviation ($\sigma\zeta$) is given as $\sigma\zeta = 0.5 \times \ln(X2/X1)$, wherein X1 and X2 are particle diameters for R=84.1% by weight and R=15.9% by weight.

The proportion of particles with a particle size of at least 150 $\mu$m is preferably at least 90% by weight, more preferably at least 95% by weight, most preferably at least 98% by weight.

Polymer particles with too small particle size lower the saline flow conductivity (SFC). The proportion of excessively small polymer particles (fines) should therefore be small.

Excessively small polymer particles are therefore typically removed and recycled into the process. This is preferably done before, during or immediately after the polymerization, i.e. before the drying of the polymer gel. The excessively small polymer particles can be moistened with water and/or aqueous surfactant before or during the recycling.

It is also possible in later process steps to remove excessively small polymer particles, for example after the surface postcrosslinking or another coating step. In this case, the excessively small polymer particles recycled are surface postcrosslinked or coated in another way, for example with fumed silica.

When a kneading reactor is used for polymerization, the excessively small polymer particles are preferably added during the last third of the polymerization.

When the excessively small polymer particles are added at a very early stage, for example actually to the monomer solution, this lowers the centrifuge retention capacity (CRC) of the resulting fluid-absorbing polymer particles. However, this can be compensated, for example, by adjusting the amount of crosslinker b) used.

When the excessively small polymer particles are added at a very late stage, for example not until an apparatus connected downstream of the polymerization reactor, for example to an extruder, the excessively small polymer particles can be incorporated into the resulting polymer gel only with difficulty. Insufficiently incorporated, excessively small polymer particles are, however, detached again from the dried polymer gel during the grinding, are therefore removed again in the course of classification and increase the amount of excessively small polymer particles to be recycled.

The proportion of particles having a particle size of at most 850 $\mu$m is preferably at least 90% by weight, more preferably at least 95% by weight, most preferably at least 98% by weight.

The proportion of particles having a particle size of at most 600 $\mu$m is preferably at least 90% by weight, more preferably at least 95% by weight, most preferably at least 98% by weight.

Polymer particles with too great particle size lower the swell rate. The proportion of excessively large polymer particles should therefore likewise be small.

Excessively large polymer particles are therefore typically removed and recycled into the grinding of the dried polymer gel.

To further improve the properties, the polymer particles can be surface postcrosslinked. Suitable surface postcrosslinkers are compounds which comprise groups which can form covalent bonds with at least two carboxylate groups of the polymer particles. Suitable compounds are, for example, polyfunctional amines, polyfunctional amidoamines, polyfunctional epoxides, as described in EP 0 083 022 A2, EP 0 543 303 A1 and EP 0 937 736 A2, di- or polyfunctional alcohols, as described in DE 33 14 019 A1, DE 35 23 617 A1 and EP 0 450 922 A2, or $\beta$-hydroxyalkylamides, as described in DE 102 04 938 A1 and US 6,239,230.

Additionally described as suitable surface postcrosslinkers are cyclic carbonates in DE 40 20 780 C1, 2-oxazolidone and its derivatives, such as 2-hydroxyethyl-2-oxazolidone in DE 198 07 502 A1, bis- and poly-2-oxazolidinones in DE 198 07 992 C1, 2-oxotetrahydro-1,3-oxazine and its derivatives in DE 198 54 573 A1, N-acyl-2-oxazolidones in DE 198 54 574 A1, cyclic ureas in DE 102 04 937 A1, bicyclic amide acetals in DE 103 34 584 A1, oxetanes and cyclic ureas in EP 1 199 327 A2 and morpholine-2,3-dione and its derivatives in WO 2003/031482 A1.

Preferred surface postcrosslinkers are ethylene carbonate, ethylene glycol diglycidyl ether, reaction products of polyamides with epichlorohydrin, and mixtures of propylene glycol and 1,4-butanediol.

Very particularly preferred surface postcrosslinkers are 2-hydroxyethyloxazolidin-2-one, oxazolidin-2-one and 1,3-propanediol.

In addition, it is also possible to use surface postcrosslinkers which comprise additional polymerizable ethylenically unsaturated groups, as described in DE 37 13 601 A1.

The amount of surface postcrosslinkers is preferably 0.001 to 2% by weight, more preferably 0.02 to 1% by weight, most preferably 0.05 to 0.2% by weight, based in each case on the polymer particles.

In a preferred embodiment, polyvalent cations are applied to the particle surface in addition to the surface postcrosslinkers before, during or after the surface postcrosslinking.

The polyvalent cations usable are, for example, divalent cations such as the cations of zinc, magnesium, calcium and strontium, trivalent cations such as the cations of aluminum, tetravalent cations such as the cations of titanium and zirconium. Possible counterions are, for example, chloride, bromide, sulfate, hydrogensulfate, carbonate, hydrogencarbonate, nitrate, phosphate, hydrogenphosphate, dihydrogenphosphate and carboxylate, such as acetate and lactate. Aluminum sulfate and aluminum lactate are preferred. Apart from metal salts, it is also possible to use polyamines as polyvalent cations. A single metal salt can be used as well as any mixture of the metal salts and/or the polyamines above.

The amount of polyvalent cation used is, for example, 0.001 to 1.5% by weight, preferably 0.005 to 1% by weight, more preferably 0.02 to 0.8% by weight, based in each case on the polymer particles.

The surface postcrosslinking is typically performed in such a way that a solution of the surface postcrosslinker is sprayed onto the dried polymer particles. After the spraying, the polymer particles coated with surface postcrosslinker are dried thermally, and the surface postcrosslinking reaction can take place either before or during the drying.

The spraying of a solution of the surface postcrosslinker is preferably performed in mixers with moving mixing tools, such as screw mixers, disk mixers and paddle mixers. Particular preference is given to horizontal mixers such as paddle mixers, very particular preference to vertical mixers. The distinction between horizontal mixers and vertical mixers is made by the position of the mixing shaft, i.e. horizontal mixers have a horizontally mounted mixing shaft and vertical mixers a vertically mounted mixing shaft. Suitable mixers are, for example, horizontal Pflugschar® plowshare mixers (Gebr. Lödige Maschinenbau GmbH; Paderborn; Germany), Vrieco-Nauta continuous mixers (Hosokawa Micron BV;

Doetinchem; the Netherlands), Processall Mixmill mixers (Proces-sall Incorporated; Cincinnati; US) and Schugi Flexomix® (Hosokawa Micron BV; Doetinchem; the Netherlands). However, it is also possible to spray on the surface postcrosslinker solution in a fluidized bed.

The surface postcrosslinkers are typically used in the form of an aqueous solution. The content of nonaqueous solvent and/or total amount of solvent can be used to adjust the penetration depth of the surface postcrosslinker into the polymer particles.

When exclusively water is used as the solvent, a surfactant is advantageously added. This improves the wetting performance and reduces the tendency to form lumps. However, preference is given to using solvent mixtures, for example isopropanol/water, 1,3-propanediol/water and propylene glycol/water, where the mixing ratio by mass is preferably from 20:80 to 40:60.

The thermal drying is preferably carried out in contact dryers, more preferably paddle dryers, most preferably disk dryers. Suitable driers are, for example, Hosokawa Bepex® horizontal paddle driers (Hosokawa Micron GmbH; Leingarten; Germany), Hosokawa Bepex® disk driers (Hosokawa Micron GmbH; Leingarten; Germany), Holo-Flite® dryers (Metso Minerals Industries Inc.; Danville; U.S.A.) and Nara paddle driers (NARA Machinery Europe; Frechen; Germany). Nara paddle driers and, in the case of using polyfunctional epoxides, Holo-Flite® dryers are preferred. Moreover, it is also possible to use fluidized bed dryers.

The drying can be effected in the mixer itself, by heating the jacket or blowing in warm air. Equally suitable is a downstream drier, for example a shelf drier, a rotary tube oven or a heatable screw. It is particularly advantageous to mix and dry in a fluidized bed dryer.

Preferred drying temperatures are in the range of 100 to 250°C, preferably 120 to 220°C, more preferably 130 to 210°C, most preferably 150 to 200°C. The preferred residence time at this temperature in the reaction mixer or drier is preferably at least 10 minutes, more preferably at least 20 minutes, most preferably at least 30 minutes, and typically at most 60 minutes.

It is preferable to cool the polymer particles after thermal drying. The cooling is preferably carried out in contact coolers, more preferably paddle coolers, most preferably disk coolers. Suitable coolers are, for example, Hosokawa Bepex® horizontal paddle coolers (Hosokawa Micron GmbH; Leingarten; Germany), Hosokawa Bepex® disk coolers (Hosokawa Micron GmbH; Leingarten; Germany), Holo-Flite® coolers (Metso Minerals Industries Inc.; Danville; U.S.A.) and Nara paddle coolers (NARA Machinery Europe; Frechen; Germany). Moreover, it is also possible to use fluidized bed coolers.

In the cooler the polymer particles are cooled to temperatures of in the range from 20 to 150°C, preferably from 40 to 120°C, more preferably from 60 to 100°C, most preferably from 70 to 90°C. Cooling using warm water is preferred, especially when contact coolers are used.

Subsequently, the surface postcrosslinked polymer particles can be classified again, excessively small and/or excessively large polymer particles being removed and recycled into the process.

To further improve the properties, the surface postcrosslinked polymer particles can be coated and/or remoisturized.

Suitable coatings for controlling the acquisition behavior and improving the permeability (SFC or GBP) are, for example, inorganic inert substances, such as water-insoluble metal salts, organic polymers, cationic polymers and polyvalent metal cations. Suitable coatings for improving the color stability are, for example reducing agents and antioxidants. Suitable coatings for dust binding are, for example, polyols. Suitable coatings against the undesired caking tendency of the polymer particles are, for example, fumed silica, such as Aerosil® 200, and surfactants, such as Span® 20. Preferred coatings are aluminium monoacetate, aluminium sulfate, aluminium lactate, Bruggolite® FF7 and Span® 20.

Suitable inorganic inert substances are silicates such as montmorillonite, kaolinite and talc, zeolites, activated carbons, polysilicic acids, magnesium carbonate, calcium carbonate, calcium phosphate, barium sulfate, aluminum oxide, titanium dioxide and iron(II) oxide. Preference is given to using polysilicic acids, which are divided between precipitated silicas and fumed silicas according to their mode of preparation. The two variants are commercially available under the names Silica FK, Sipernat®, Wessalon® (precipitated silicas) and Aerosil® (fumed silicas) respectively. The inorganic inert substances may be used as dispersion in an aqueous or water-miscible dispersant or in substance.

When the fluid-absorbent polymer particles are coated with inorganic inert substances, the amount of inorganic inert substances used, based on the fluid-absorbent polymer particles, is preferably from 0.05 to 5% by weight, more preferably from 0.1 to 1.5% by weight, most preferably from 0.3 to 1 % by weight.

Suitable organic polymers are polyalkyl methacrylates or thermoplastics such as polyvinyl chloride, waxes based on polyethylene, polypropylene, polyamides or polytet-rafluoro-ethylene. Other examples are styrene-isoprene-styrene block-copolymers or styrene-butadiene-styrene block-copolymers.

Suitable cationic polymers are polyalkylenepolyamines, cationic derivatives of polyacrylamides, polyethyleneimines and polyquaternary amines.

Polyquaternary amines are, for example, condensation products of hexamethylenediamine dimethylamine and epichlorohydrin, condensation products of dimethylamine and epichlorohydrin, copolymers of hydroxyethylcellulose and diallyldimethylammonium chloride, copolymers of acrylamide and α-methacryloyloxyethyltrimethaylammonium chloride, condensation products of hydroxyethylcellulose, epichlorohydrin and trimethylamine, homopolymers of diallyldimethylammonium chloride and addition products of epichlorohydrin to amidoamines. In addition, polyquaternary amines can be obtained by reacting dimethyl sulfate with polymers such as polyethyleneimines, copolymers of vinylpyrrolidone and dimethylaminoethyl methacrylate or copolymers of ethyl methacrylate and diethylaminoethyl methacrylate. The polyquaternary amines are available within a wide molecular weight range.

However, it is also possible to generate the cationic polymers on the particle surface, either through reagents which can form a network with themselves, such as addition products of epichlorohydrin to polyamidoamines, or through the application of cationic polymers which can react with an added crosslinker, such as polyamines or polyimines in combination with polyepoxides, polyfunctional esters, polyfunctional acids or poly-functional (meth)acrylates.

It is possible to use all polyfunctional amines having primary or secondary amino groups, such as polyethyleneimine, polyallylamine and polylysine. The liquid sprayed by the process according to the invention preferably comprises at least one polyamine, for example polyvinylamine or a partially hydrolyzed polyvinylformamide.

The cationic polymers may be used as a solution in an aqueous or water-miscible solvent, as dispersion in an aqueous or water-miscible dispersant or in substance.

When the fluid-absorbent polymer particles are coated with a cationic polymer, the use amount of cationic polymer based on the fluid-absorbent polymer particles is usually not less than 0.001% by weight, typically not less than 0.01% by weight, preferably from 0.1 to 15% by weight, more preferably from 0.5 to 10% by weight, most preferably from 1 to 5% by weight.

Suitable polyvalent metal cations are Mg2+, Ca2+, Al3+, Sc3+, Ti4+, Mn2+, Fe2+/3+, Co2+, Ni2+, Cu+/2+, Zn2+, Y3+, Zr4+, Ag+, La3+, Ce4+, Hf4+ and Au+/3+; preferred metal cations are Mg2+, Ca2+, Al3+, Ti4+, Zr4+ and La3+; particularly preferred metal cations are Al3+, Ti4+ and Zr4+. The metal cations may be used either alone or in a mixture with one another. Suitable metal salts of the metal cations mentioned are all of those which have a sufficient solubility in the solvent to be used. Particularly suitable metal salts have weakly complexing anions, such as chloride, hydroxide, carbonate, nitrate and sulfate. The metal salts are preferably used as a solution or as a stable aqueous colloidal dispersion. The solvents used for the metal salts may be water, alcohols, dimethylformamide dimethyl sulfoxide and mixtures thereof. Particular preference is given to water and water/alcohol mixtures, such as water/methanol, water/isopropanol, water/1,3-propanediole, water/1,2-propandiole/1,4-butanediole or water/propylene glycol.

When the fluid-absorbent polymer particles are coated with a polyvalent metal cation, the amount of polyvalent metal cation used, based on the fluid-absorbent polymer particles, is preferably from 0.05 to 5% by weight, more preferably from 0.1 to 1.5% by weight, most preferably from 0.3 to 1 % by weight.

Suitable reducing agents are, for example, sodium sulfite, sodium hydrogensulfite (sodium bisulfite), sodium dithionite, sulfinic acids and salts thereof, ascorbic acid, sodium hypophosphite, sodium phosphite, and phosphinic acids and salts thereof. Preference is given, however, to salts of hypophosphorous acid, for example sodium hyprophosphite salts of sulfinic acids, for example the disodium salt of 2-hydroxy-2-sulfinato-acetic acid, and addition products of aldehydes, for example the disodium salt of 2-hydroxy-2-sulfonatoacetic acid. The reducing agent used can be, however, a mixture of the sodium salt of 2-hydroxy-2-sulfinatoacetic acid, the disodium salt of 2-hydroxy-2-sulfonatoacetic acid and sodium bisulfite. Such mixtures are obtainable as Brüggolite® FF6 and Brüggolite® FF7 (Brüggemann Chemicals; Heilbronn; Germany).

The reducing agents are typically used in the form of a solution in a suitable solvent, preferably water. The reducing agent may be used as a pure substance or any mixture of the above reducing agents may be used.

When the fluid-absorbent polymer particles are coated with a reducing agent, the amount of reducing agent used, based on the fluid-absorbent polymer particles, is preferably from 0.01 to 5% by weight, more preferably from 0.05 to 2% by weight, most preferably from 0.1 to 1% by weight.

Suitable polyols are polyethylene glycols having a molecular weight of from 400 to 20000 g/mol, polyglycerol, 3- to 100-tuply ethoxylated polyols, such as trimethylolpropane, glycerol, sorbitol and neopentyl glycol. Particularly suitable polyols are 7- to 20-tuply ethoxylated glycerol or trimethylolpropane, for example Polyol TP 70® (Perstorp AB, Perstorp, Sweden). The latter have the advantage in particular that they lower the surface tension of an aqueous extract of the fluid-absorbent polymer particles only insignificantly. The polyols are preferably used as a solution in aqueous or water-miscible solvents.

When the fluid-absorbent polymer particles are coated with a polyol, the use amount of polyol, based on the fluid-absorbent polymer particles, is preferably from 0.005 to 2% by weight, more preferably from 0.01 to 1 % by weight, most preferably from 0.05 to 0.5% by weight.

The coating is preferably performed in mixers with moving mixing tools, such as screw mixers, disk mixers, paddle mixers and drum coater. Suitable mixers are, for example, horizontal Pflugschar® plowshare mixers (Gebr. Lödige Maschinenbau GmbH; Paderborn; Germany), Vrieco-Nauta Continuous Mixers (Hosokawa Micron BV; Doetinchem; the Netherlands), Processall Mixmill Mixers (Processall Incorporated; Cincinnati; US) and Ruberg continuous flow mixers (Gebrüder Ruberg GmbH & Co KG, Nieheim, Germany). Moreover, it is also possible to use a fluidized bed for mixing.

The fluid-absorbing polymer particles usable for the fluid-absorbent cores according to the invention have a centrifuge retention capacity (CRC) of typically at least 24 g/g, preferably at least 26 g/g, preferentially at least 28 g/g, more preferably at least 30 g/g. The centrifuge retention capacity (CRC) of the fluid-absorbing polymer particles is typically less than 60 g/g. The centrifuge retention capacity (CRC) is determined by EDANA recommended test method No. WSP 241.2-05 "Centrifuge Retention Capacity".

The fluid-absorbing polymer particles usable for the fluid-absorbent cores according to the invention have an absorbency under a load of 49.2 $g/cm^2$ of typically at least 18 g/g, preferably at least 20 g/g, more preferably at least 22 g/g, most preferably at least 25 g/g. The absorbency under a load of 49.2 $g/cm^2$ of the fluid-absorbing polymer particles is typically less than 35 g/g. The absorbency under a load of 49.2 $g/cm^2$ is determined analogously to EDANA recommended test method No. WSP 242.2-05 "Absorption under Pressure", except that a pressure of 49.2 $g/cm^2$ is established instead of a pressure of 21.0 $g/cm^2$.

The fluid-absorbent polymer particles useable for the fluid-absorbent cores according to the invention have a saline flow conductivity (SFC) of typically at least 20 x 10 -7 cm3 s/g, preferably at least 25 x 10 -7 cm3 s/g, preferentially preferably at least 30 x 10 -7 cm3 s/g, most preferably at least 50 x 10 -7 cm3 s/g. The saline flow conductivity (SFC) of the fluid-absorbent polymer particles is typically less than 500 x 10 -7 cm3 s/g. The saline flow conductivity is basically determined according to EP 0 640 330 A1, as the gel layer permeability of a swollen gel layer of fluid-absorbent polymer particles.

The fluid-absorbent polymer particles useable for the fluid-absorbent cores according to the invention have a free swell gel bed permeability (GBP) of typically at least 20 Darcies, preferably at least 50 Darcies, preferentially at least 70 Darcies, more preferably at least 90 Darcies, most preferably at least 100 Darcies, and typically not more than 250 Darcies. The method to determine the free swell gel bed permeability is described in US 2005/0256757, paragraphs [0061] to [0075].

Fluid-absorbent polymer particles suitable for the inventive fluid-absorbing articles have an apparent bulk density of preferably 0.47 to 0.78 g/cm3, more preferably 0.55 to 0.75 g/cm3, most preferably 0.60 to 0.70 g/cm3. The bulk density is determined according to EDANA test method WSP 260.2 (05).

Preferred fluid-absorbent polymer particles are polymer particles having a centrifuge retention capacity (CRC) of at least 24 g/g, and a saline flow conductivity (SFC) of at least 20x10-7 $cm^3$s/g, a bulk density of at least 0.55 $g/cm^3$ and absorbency under high load of at least 18 g/g.

C. Fluid-absorbent Articles

The fluid-absorbent article comprises of

(A) an upper liquid-pervious layer
(B) a lower liquid-impervious layer
(C) a fluid-absorbent core between (A) and (B) comprising
from 0 to 20% by weight fibrous material and from 80 to 100% by weight fluid-absorbent polymer particles;
preferably from 3 to 15% by weight fibrous material and from 85 to 97% by weight fluid-absorbent polymer particles;
more preferably from 5 to 10% by weight fibrous material and from 90 to 95% by weight fluid-absorbent polymer particles;
most preferably no fibrous material and 100 % by weight of fluid-absorbent polymer particles;
(D) an optional acquisition-distribution layer between (A) and (C), comprising from 80 to 100% by weight fibrous material and from 0 to 20% by weight fluid-absorbent polymer particles;
preferably from 85 to 99.9% by weight fibrous material and from 0.01 to 15% by weight fluid-absorbent polymer particles;
more preferably from 90 to 99.5% by weight fibrous material and from 0.5 to 10% by weight fluid-absorbent polymer particles;
most preferably from 95 to 99% by weight fibrous material and from 1 to 5% by weight fluid-absorbent polymer particles;
(E) an optional tissue layer disposed immediately above and/or below (C); and
(F) other optional components.

Fluid-absorbent articles are understood to mean, for example, incontinence pads and incontinence briefs for adults or diapers for babies. Suitable fluid-absorbent articles including fluid-absorbent compositions comprising fibrous materials and fluid-absorbent polymer particles to form fibrous webs or matrices for the substrates, layers, sheets and/or the fluid-absorbent core.

Suitable fluid-absorbent articles may be composed of several layers whose individual elements must show preferably definite functional parameter such as dryness for the upper liquid-pervious layer, vapor permeability without wetting through for the lower liquid-impervious layer, a flexible, vapor permeable and thin fluid-absorbent core, showing fast absorption rates and being able to retain highest quantities of body fluids, and an acquisition-distribution layer between the upper layer and the core, acting as transport and distribution layer of the discharged body fluids. These individual elements are combined such that the resultant fluid-absorbent article meets overall criteria such as flexibility, water vapor breathability, dryness, wearing comfort and protection on the one side, and concerning liquid retention, rewet and prevention of wet through on the other side. The specific combination of these layers provides a fluid-absorbent article delivering both high protection levels as well as high comfort to the consumer.

Liquid-pervious Layer (A)

The liquid-pervious layer (A) is the layer which is in direct contact with the skin. Thus, the liquid-pervious layer is preferably compliant, soft feeling and non-irritating to the consumer's skin. Generally, the term "liquid-pervious" is understood thus permitting liquids, i.e. body fluids such as urine, menses and/or vaginal fluids to readily penetrate through its thickness. The principle function of the liquid-pervious layer is the acquisition and transport of body fluids from the wearer towards the fluid-absorbent core. Typically liquid-pervious layers are formed from any materials known in the art such as nonwoven material, films or combinations thereof. Suitable liquid-pervious layers (A) consist of customary synthetic or semisynthetic fibers or bicomponent fibers or films of polyester, polyolefins, rayon or natural fibers or any combinations thereof. In the case of nonwoven materials, the fibers should generally be bound by binders such as polyacrylates. Additionally the liquid-pervious layer may contain elastic compositions thus showing elastic characteristics allowing to be stretched in one or two directions.

Suitable synthetic fibers are made from polyvinyl chloride, polyvinyl fluoride, polytetrafluorethylene, polyvinylidene chloride, polyacrylics, polyvinyl acetate, polyethylvinyl acetate, non-soluble or soluble polyvinyl alcohol, polyolefins such as polyethylene, polypropylene, polyamides, polyesters, polyurethanes, polystyrenes and the like.

Examples for films are apertured formed thermoplastic films, apertured plastic films, hydroformed thermoplastic films, reticulated thermoplastic films, porous foams, reticulated foams, and thermoplastic scrims.

Examples of suitable modified or unmodified natural fibers include cotton, bagasse, kemp, flax, silk, wool, wood pulp, chemically modified wood pulp, jute, rayon, ethyl cellulose, and cellulose acetate.

Suitable wood pulp fibers can be obtained by chemical processes such as the Kraft and sulfite processes, as well as from mechanical processes, such as ground wood, refiner mechanical, thermo-mechanical, chemi-mechanical and chemi-thermo-mechanical pulp processes. Further, recycled wood pulp fibers, bleached, unbleached, elementally chlorine free (ECF) or total chlorine free (TCF) wood pulp fibers can be used.

The fibrous material may comprise only natural fibers or synthetic fibers or any combination thereof. Preferred materials are polyester, rayon and blends thereof, polyethylene, and polypropylene.

The fibrous material as a component of the fluid-absorbent compositions may be hydrophilic, hydrophobic or can be a combination of both hydrophilic and hydrophobic fibers. The definition of hydrophilic is given in the section "definitions" in the chapter above. The selection of the ratio hydrophilic/hydrophobic and accordingly the amount of hydrophilic and hydrophobic fibers within fluid-absorbent composition will depend upon fluid handling properties and the amount of fluid-absorbent polymer particles of the resulting fluid-absorbent composition. Such, the use of hydrophobic fibers is preferred if the fluid-absorbent composition is adjacent to the wearer of the fluid-absorbent article, that is to be used to replace partially or completely the upper liquid-pervious layer, preferably formed from hydrophobic nonwoven materials. Hydrophobic fibers can also be member of the lower breathable, but fluid-impervious layer, acting there as a fluid-impervious barrier.

Examples for hydrophilic fibers are cellulosic fibers, modified cellulosic fibers, rayon, polyester fibers such as polyethylen terephthalate, hydrophilic nylon and the like. Hydrophilic fibers can also be obtained from hydrophobic fibers which are hydrophilized by e. g. surfactant-treating or silica-treating. Thus, hydrophilic thermoplastic fibers derived from polyolefins such as polypropylene, polyamides, polystyrenes or the like by surfactant-treating or silica-treating.

To increase the strength and the integrity of the upper-layer, the fibers should generally show bonding sites, which act as crosslinks between the fibers within the layer.

Technologies for consolidating fibers in a web are mechanical bonding, thermal bonding and chemical bonding. In the process of mechanical bonding the fibers are entangled mechanically, e.g., by water jets (spunlace) to give integrity to the web. Thermal bonding is carried out by means of rising the temperature in the presence of low-melting polymers. Examples for thermal bonding processes are spunbonding, through-air bonding and resin bonding.

Preferred means of increasing the integrity are thermal bonding, spunbonding, resin bonding, through-air bonding and/or spunlace.

In the case of thermal bonding, thermoplastic material is added to the fibers. Upon thermal treatment at least a portion of this thermoplastic material is melting and migrates to intersections of the fibers caused by capillary effects. These intersections solidify to bond sites after cooling and increase the integrity of the fibrous matrix. Moreover, in the case of chemically stiffened cellulosic fibers, melting and migration of the thermoplastic material has the effect of increasing the pore size of the resultant fibrous layer while maintaining its density and basis weight. Upon wetting, the structure and integrity of the layer remains stable. In summary, the addition of thermoplastic material leads to improved fluid permeability of discharged body fluids and thus to improved acquisition properties.

Suitable thermoplastic materials including polyolefins such as polyethylene and polypropylene, polyesters, copolyesters, polyvinyl acetate, polyethylvinyl acetate, polyvinyl chloride, polyvinylidene chloride, polyacrylics, polyamides, copolyamides, polystyrenes, polyurethanes and copolymers of any of the mentioned polymers.

Suitable thermoplastic fibers can be made from a single polymer that is a mono-component fiber. Alternatively, they can be made from more than one polymer, e.g., bicom-ponent or multicomponent fibers. The term "bicomponent fibers" refers to thermoplastic fibers that comprise a core fiber made from a different fiber material than the shell. Typically, both fiber materials have different melting points, wherein generally the sheath melts at lower temperatures. Bi-component fibers can be concentric or eccentric depending whether the sheath has a thickness that is even or uneven through the cross-sectional area of the bi-component fiber. Advantage is given for eccentric bi-component fibers showing a higher compressive strength at lower fiber thickness. Further bi-component fibers can show the feature "uncrimped" (unbent) or "crimped" (bent), further bi-component fibers can demonstrate differing aspects of surface lubricity.

Examples of bi-component fibers include the following polymer combinations: polyethylene/polypropylene, polyethylvinyl acetate/polypropylene, polyethylene/polyester, polypropylene/polyester, copolyester/polyester and the like.

Suitable thermoplastic materials have a melting point of lower temperatures that will damage the fibers of the layer; but not lower than temperatures, where usually the fluid-absorbent articles are stored. Preferably the melting point is between about 75°C and 175°C. The typical length of thermoplastic fibers is from about 0.4 to 6 cm, preferably from about 0.5 to 1 cm. The diameter of thermoplastic fibers is defined in terms of either denier (grams per 9000 meters) or dtex (grams per 10 000 meters). Typical thermoplastic fibers have a dtex in the range from about 1.2 to 20, preferably from about 1.4 to 10.

A further mean of increasing the integrity of the fluid-absorbent composition is the spunbonding technology. The nature of the production of fibrous layers by means of spunbonding is based on the direct spinning of polymeric granulates into continuous filaments and subsequently manufacturing the fibrous layer.

Spunbond fabrics are produced by depositing extruded, spun fibers onto a moving belt in a uniform random manner followed by thermal bonding the fibers. The fibers are separated during the web laying process by air jets. Fiber bonds are generated by applying heated rolls or hot needles to partially melt the polymer and fuse the fibers together. Since molecular orientation increases the melting point, fibers that are not highly drawn can be used as thermal binding fibers. Polyethylene or random ethylene/ propylene copolymers are used as low melting bonding sites.

Besides spunbonding, the technology of resin bonding also belongs to thermal bonding subjects. Using this technology to generate bonding sites, specific adhesives, based on e.g. epoxy, polyurethane and acrylic are added to the fibrous material and the resulting matrix is thermal treated. Thus the web is bonded with resin and/or thermal plastic resins dispersed within the fibrous material.

As a further thermal bonding technology through-air bonding involves the application of hot air to the surface of the fibrous fabric. The hot air is circulated just above the fibrous fabric, but does not push through the fibrous fabric. Bonding sites are generated by the addition of binders. Suitable binders used in through-air thermal bonding include crystalline binder fibers, bi-component binder fibers, and powders. When using crystalline binder fibers or powders, the binder melts entirely and forms molten droplets throughout the nonwoven's cross-section. Bonding occurs at these points upon cooling. In the case of sheath/core binder fibers, the sheath is the binder and the core is the carrier fiber. Products manufactured using through-air ovens tend to be bulky, open, soft, strong, extensible, breathable and absorbent. Through-air bonding followed by immediate cold calendering results in a thickness between a hot roll calendered product and one that has been though-air bonded without compression. Even after cold calendering, this product is softer, more flexible and more extensible than area-bond hot-calendered material.

Spunlacing ("hydroentanglement") is a further method of increasing the integrity of a web. The formed web of loose fibers (usually air-laid or wet-laid) is first compacted and prewetted to eliminate air pockets. The technology of spunlacing uses multiple rows of fine high-speed jets of water to strike the web on a porous belt or moving perforated or patterned screen so that the fibers knot about one another. The water pressure generally increases from the first to the last injectors. Pressures as high as 150 bar are used to direct the water jets onto the web. This pressure is sufficient for most of the nonwoven fibers, although higher pressures are used in specialized applications.

The spunlace process is a nonwoven manufacturing system that employs jets of water to entangle fibers and thereby provide fabric integrity. Softness, drape, conformability, and relatively high strength are the major characteristics of spunlace nonwoven.

In newest researches benefits are found in some structural features of the resulting liquid-pervious layers. For example, the thickness of the layer is very important and influences together with its x-y dimension the acquisition-distribution behavior of the layer. If there is further some profiled structure integrated, the acquisition-distribution behavior can be directed depending on the three-dimensional structure of the layer. Thus 3D-polyethylene in the function of liquid-pervious layer is preferred.

Thus, suitable liquid-pervious layers (A) are nonwoven layers formed from the fibers above by thermal bonding, spunbonding, resin bonding or through-air bonding. Further suitable liquid-pervious layers are 3D-polyethylene layers and spunlace.

Preferably the 3D-polyethylene layers and spunlace show basis weights from 12 to 22 gsm.

Typically liquid- pervious layers (A) extend partially or wholly across the fluid-absorbent structure and can extend into and/or form part of all the preferred sideflaps, side wrapping elements, wings and ears.

Liquid-impervious Layer (B)

The liquid-impervious layer (B) prevents the exudates absorbed and retained by the fluid-absorbent core from wetting articles which are in contact with the fluid-absorbent article, as for example bedsheets, pants, pyjamas and undergarments. The liquid-impervious layer (B) may thus comprise a woven or a nonwoven material, polymeric films such as thermoplastic film of polyethylene or polypropylene, or composite materials such as film-coated nonwoven material.

Suitable liquid-impervious layers include nonwoven, plastics and/or laminates of plastic and nonwoven. Both, the plastics and/or laminates of plastic and nonwoven may appropriately be breathable, that is, the liquid-impervious layer (B) can permit vapors to escape from the fluid-absorbent material. Thus the liquid-impervious layer has to have a definite water vapor transmission rate and at the same time the level of impermeability. To combine these features, suitable liquid-impervious layers including at least two layers, e.g. laminates from fibrous nonwoven having a specified basis weight and pore size, and a continuous three-dimensional film of e.g. polyvinylalcohol as the second layer having a specified thickness and optionally having pore structure. Such laminates acting as a barrier and showing no liquid transport or wet through. Thus, suitable liquid-impervious layers comprising at least a first breathable layer of a porous web which is a fibrous nonwoven, e.g. a composite web of a meltblown nonwoven layer or of a spunbonded nonwoven layer made from synthetic fibers and at least a second layer of a resilient three dimensional web consisting of a liquid-impervious polymeric film, e.g. plastics optionally having pores acting as capillaries, which are preferably not perpendicular to the plane of the film but are disposed at an angle of less than 90° relative to the plane of the film.

Suitable liquid-impervious layers are permeable for vapor. Preferably the liquid-impervious layer is constructed from vapor permeable material showing a water vapor transmission rate (WVTR) of at least about 100 gsm per 24 hours, preferably at least about 250 gsm per 24 hours and most preferred at least about 500 gsm per 24 hours.

Preferably the liquid-impervious layer (B) is made of nonwoven comprising hydrophobic materials, e.g. synthetic fibers or a liquid-impervious polymeric film comprising plastics e.g. polyethylene. The thickness of the liquid-impervious layer is preferably 15 to 30 $\mu$m.

Further, the liquid-impervious layer (B) is preferably made of an absorbent core of nonwoven and plastics comprising a nonwoven having a density of 12 to 15 gsm and a polyethylene layer having a thickness of about 10 to 20 $\mu$m.

The typically liquid-impervious layer (B) extends partially or wholly across the fluid-absorbent structure and can extend into and/or form part of all the preferred sideflaps, side wrapping elements, wings and ears.

Fluid-absorbent Core (C)

The fluid-absorbent core (C) usually is disposed between the upper liquid-pervious layer (A) and the lower liquid-impervious layer (B).

The absorbent core may be available in a core wrap, i.e. an envelope around the absorbing material. This core wrap or envelope typically comprises a bottom layer and a top layer, where the bottom layer contacts the lower layer (B) and the top layer contacts the upper layer (A). It is also possible that the bottom layer be omitted and the lower layer (B) then plays both roles of lower liquid-impervious layer (B) per se and bottom layer. It is also possible that the top layer be omitted and the upper liquid-pervious layer (A) then plays both roles of upper liquid-pervious layer (A) per se and top layer.

The top view area of the fluid-absorbent core (C) is preferably at least 200cm$^2$, more preferably at least 250cm$^2$, most preferably at least 300cm$^2$. The top view area is the part of the core that is face-to-face to the upper liquid-pervious layer.

Typically the fluid-absorbent cores may contain a single type of fluid-absorbent polymer particles or may contain fluid-absorbent polymer particles derived from different kinds of fluid-absorbent polymer material. Thus, it is possible to add fluid-absorbent polymer particles from a single kind of polymer material or a mixture of fluid-absorbent polymer particles from different kinds of polymer materials, e.g. a mixture of regular fluid-absorbent polymer particles, derived from gel polymerization with fluid-absorbent polymer particles, derived from dropletization polymerization. Alternatively it is possible to add fluid-absorbent polymer particles derived from inverse suspension polymerization.

According to the invention the absorbent cores comprises a plurality of pockets for immobilizing the fluid-absorbent particles.

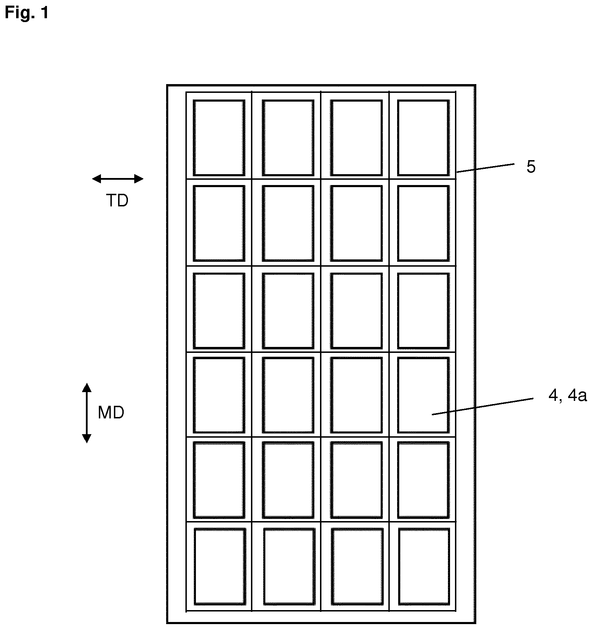

Reference is made to figure 1 which shows a top view of an adsorbent core with pockets in rectangular shape and beads in machine direction (MD) and transverse direction (TD). A two sheet layer (2) which may serve as the top and bottom layer. In one embodiment of the invention at least one of the layers may be coated with a layer of adhesive, typically a hot-melt. The adhesive may be present on the entire surface or only in defined areas e.g. stripes or only at the vicinity of the pockets (4,4a). The adhesive receive the SAP and adhere to it so that most of the SAP will be caused to adhere to the surface of the sheet layer in the pocket regions. To form the shape of the pocket (4, 4a) e.g. a vacuum may be applied to the layer.

In addition, e.g. to improve the core integrity adhesive beads (5) (also known as adhesive ropes) may be placed between the pockets (4, 4a), as illustrated in Fig. 1.

The pockets can have various shapes and forms. For example, the pockets can be rectangular or square in shape with varying lengths of their respective sides. For example the length may vary from 10mmx10mm to 10mmx80mm, including 20mmx20mm to 20mmx60mm or 20mmx20mm to 40mmx40mm or 20mmx40mm with varying shapes, in any direction. The depth of the final pocket depends e. g. on the mass of SAP material to be filled in. For example for baby diapers a depth from 1 mm to 5mm (once finally formed, i.e. pleated or calendered) may be preferred. Any other desired geometric forms and patterns are conceivable.

The pockets may assume any desired shape in terms of area, for example circles, ellipses, rectangles, squares, triangles (viewed from above). Particular preference is given to any desired polygons or mixtures of polygons.

Particular preference is also given to the application of one or more continuous strips in machine direction (MD), the strips running parallel to one another.

Furthermore it is preferred that the adhesive beads are forming a connected network surrounding the pockets.

The fluid-absorbent particles are applied to the pockets e.g. by dosing devices, so that the particle size distribution of the polymer will not differ between different pockets of the absorbent core by more than 15%, e.g. by dosing the particles into a plurality of particle flows using a housing with a rotating mobile imparting a rotation to the fluid-absorbent particles, wherein the flows being substantially of similar flow rates, notably according to application filed under EP appln 12199197.0, entitled "NOVEL PROCESS FOR DOSING SUPER ABSORBENT PARTICLES", or e.g. by dosing the particles using individual dosing devices having substantially identical parameters to deliver the required particle size distribution.

A second layer, e.g. a water-permeable one so as to allow the fluids to penetrate through and reach the SAP may serve as the top layer.

The second layer, which optionally comprises also an adhesive layer, preferably a not continuous one is then affixed onto the first sheet layer with the pockets (4, 4a) containing the SAP and bearing the beads. The areas of junction itself may be of rectangular, circular or squared shape with diameters between e.g. about 0.5 mm and 2 mm. Fluid-absorbent articles comprising areas of junction show a better wet strength. Calendering may then performed on the sandwich thus formed, ensuring the bonding of the two sheet layers. After this pleating (or compacting) may be finally performed, so as to create pleats and tightly contain the SAP in the pockets for example for further fixation of the SAP in the pockets. This pleating step may be omitted if no pleat is required. Pleats are usually formed when the mass of SAP after calendering, but before pleating, is not in contact over substantially all its upper surface with the top layer and/or the SAP mass represents, in volume, less than 70%, preferably less than 50% of the volume defined by the pockets before pleating.

Instead of applying adhesive beads especially to join the layers as described above, the layers may be joined to each other also by mechanical, thermal or ultrasonic bonding or combinations thereof or combinations thereof with adhesives. The areas of junction may have a regular or irregular pattern, e.g. aligned with the longitudinal axis of the fluid-absorbent core or in a pattern of polygons, e.g. pentagons or hexagons. The areas of junction itself may have diameters between about 0.5 mm and 2 mm.

Fluid-absorbent cores may also comprise fibrous material to a maximum of 20% by weight.

Typically the fluid-absorbent cores may contain a single type of fluid-absorbent polymer particles or may contain fluid-absorbent polymer particles derived from different kinds of fluid-absorbent polymer material. Thus, it is possible to add fluid-absorbent polymer particles from a single kind of polymer material or a mixture of fluid-absorbent polymer particles from different kinds of polymer materials, e.g. a mixture of regular fluid-absorbent polymer particles, derived from gel polymerization with fluid-absorbent polymer particles, derived from dropletization polymerization. Alternatively it is possible to add fluid-absorbent polymer particles derived from inverse suspension polymerization.

Alternatively it is possible to mix fluid-absorbent polymer particles showing different feature profiles. Thus, the fluid-absorbent core may contain fluid-absorbent polymer particles with uniform pH value, or it may contain fluid-absorbent polymer particles with different pH values, e.g. two- or more component mixtures from fluid-absorbent polymer particles with a pH in the range from about 4.0 to about 7.0. Preferably, applied mixtures deriving from mixtures of fluid-absorbent polymer particles got from gel polymerization or inverse suspension polymerization with a pH in the range from about 4.0 to about 7.0 and fluid-absorbent polymer particles got from drop polymerization.

Suitable fluid-absorbent cores including mixtures of fluid-absorbent polymer particles and fibrous material building matrices for the incorporation of the fluid-absorbent material. Such mixtures are formed homogenously, that is all components are mixed together to get a homogenous structure. The amount of the fluid-absorbent materials may be uniform throughout the fluid-absorbent core, or may vary, e.g. between the central region and the distal region to give a profiled core concerning the concentration of fluid-absorbent material, whereas the PSD of the fluid-absorbent particles should only differ by not more than 15% between the different pockets containing particles and fibres.

Suitable fluid-absorbent cores may also include layers, which are formed by the process of manufacturing the fluid-absorbent article. The layered structure may be formed by subsequently generating the different layers in z-direction.

Alternatively layers of other materials can be added, e.g. layers of opened or closed celled foams or perforated films. Included do also laminates of at least two layers comprise said fluid-absorbent polymer material.

Thus, suitable fluid-absorbent cores comprising from 0 to 20% by weight fibrous material and from 80 to 100% by weight fluid-absorbent polymer particles; preferably from 3 to 15% by weight fibrous material and from 85 to 97% by weight fluid-absorbent polymer particles;

more preferably from 5 to 10% by weight fibrous material and from 90 to 95% by weight fluid-absorbent polymer particles; most preferably no fibrous material and 100 % by weight of fluid-absorbent polymer particles;

The quantity of fluid-absorbent polymer particles and/or fluid-absorbent fibers within the fluid-absorbent core is from 3 to 20 g, preferably from 6 to 14 g, and from 8 to 12 g in the case of maxi-diapers, and in the case of incontinence products up to about 50 g.

Typically fluid-absorbent articles comprising at least an upper liquid-pervious layer (A), at least a lower liquid-impervious layer (B) and at least one fluid-absorbent core between the layer (A) and the layer (B) besides other optional layers. In order to increase the control of body fluid absorption and/or to increase the flexibility in the ratio weight percentages of fluid-absorbent polymer particles to fibrous matrix it may be advantageous to add one or more further fluid-absorbent cores. The addition of a second fluid-absorbent core to the first fluid-absorbent core offers more possibilities in body fluid transfer and distribution. Moreover higher quantities of discharged body fluids can be retained. Having the opportunity of combining several layers showing different fluid-absorbent polymer concentration and content, it is possible to reduce the thickness of the fluid-absorbent article to a minimum even if there are several fluid-absorbent cores included.

Suitable fluid-absorbent articles are including single or multi-core systems in any combination with other layers which are typically found in fluid-absorbent articles. Preferred fluid-absorbent articles include single- or double-core systems; most preferably fluid-absorbent articles include a single fluid-absorbent core.

These layers or foldings are preferably joined to each e.g. by addition of adhesives or by mechanical, thermal or ultrasonic bonding or combinations thereof. Fluid-absorbent polymer particles may be comprised within or between the individual layers, e.g. by forming separate fluid-absorbent polymer-layers.

Thus, according to the number of layers or the height of a voluminous core, the resulting thickness of the fluid-absorbent core will be determined. Thus, fluid-absorbent cores may be flat as one layer (plateau) or have three-dimensional profile.

Generally the upper liquid-pervious layer (A) and the lower liquid-impervious layer (B) may be shaped and sized according to the requirements of the various types of fluid-absorbent articles and to accommodate various wearer's sizes. Thus, the combination of the upper liquid-pervious layer and the lower liquid-impervious layer may have all dimensions or shapes known in the art. Suitable combinations have an hourglass shape, rectangular shape, trapezoidal shape, t- or double t-shape or showing anatomical dimensions.

Concerning odor control, perfumes and/or odor control additives are optionally added. Suitable odor control additives are all substances of reducing odor developed in carrying fluid-absorbent articles over time known in the art. Thus, suitable odor control additives are inorganic materials, such as zeolites, activated carbon, bentonite, silica, aerosile, kieselguhr, clay; chelants such as ethylenediamine tetraacetic acid (EDTA), cyclodextrins, aminopolycarbonic acids, ethylenediamine tetramethylene phosphonic acid, aminophosphate, polyfunctional aromates, N,N-disuccinic acid.

Suitable odor control additives are further antimicrobial agents such as quaternary ammonium, phenolic, amide and nitro compounds and mixtures thereof; bactericides such as silver salts, zinc salts, cetylpyridinium chloride and/or triclosan as well as surfactants having an HLB value of less than 12.

Suitable odor control additives are further compounds with anhydride groups such as maleic-, itaconic-, polymaleic- or polyitaconic anhydride, copolymers of maleic acid with C2-C8 olefins or styrene, polymaleic anhydride or copolymers of maleic anhydride with isobutene, di-isobutene or styrene, compounds with acid groups such as ascorbic, benzoic, citric, salicylic or sorbic acid and fluid-soluble polymers of monomers with acid groups, homo- or co-polymers of C3-C5 mono-unsaturated carboxylic acids.

Suitable odor control additives are further perfumes such as allyl caproate, allyl cyclohexaneacetate, allyl cyclohexanepropionate, allyl heptanoate, amyl acetate, amyl propionate, anethol, anixic aldehyde, anisole, benzaldehyde, benzyl acetate benzyl acetone, benzyl alcohole, benzyl butyrate, benzyl formate, camphene, camphor gum, laevo-carveol, cinnamyl formate, cis-jasmone, citral, citronellol and its derivatives, cuminic alcohol and its derivatives, cyclal C, dimethyl benzyl carbinol and its derivatives, dimethyl octanol and its derivatives, eucalyptol, geranyl derivatives, lavandulyl acetate ligustral, d-limonene, linalool, linalyl derivatives, menthone and its derivatives, myrcene and its derivatives, neral, nerol, p-cresol, p-cymene, orange terpenes, alpha-ponene, 4-terpineol, thymol etc.

Masking agents are also used as odor control additives. Masking agents are in solid wall material encapsulated perfumes. Preferably, the wall material comprises a fluid-soluble cellular matrix which is used for time-delay release of the perfume ingredient.

Further suitable odor control additives are transition metals such as Cu, Ag, and Zn, enzymes such as urease-inhibitors, starch, pH buffering material, chitin, green tea plant extracts, ion exchange resin, carbonate, bicarbonate, phosphate, sulfate or mixtures thereof.

Preferred odor control additives are green tea plant extracts, silica, zeolite, carbon, starch, chelating agent, pH buffering material, chitin, kieselguhr, clay, ion exchange resin, carbonate, bicarbonate, phosphate, sulfate, masking agent or mixtures thereof. Suitable concentrations of odor control additives are from about 0.5 to about 300 gsm. Newest developments propose the addition of wetness indication additives. Besides electrical monitoring the wetness in the fluid-absorbent article, wetness indication additives comprising a hot melt adhesive with a wetness indicator are known. The wetness indication additive changes the colour from yellow to a relatively dark and deep blue. This colour change is readily perceivable through the liquid-impervious outer material of the fluid-absorbent article. Existing wetness indication is also achieved via application of water soluble ink patterned on the backsheet which disappears when wet.

Suitable wetness indication additives comprising a mixture of sorbitan monooleate and polyethoxylated hydrogenated castor oil. Preferably, the amount of the wetness indication additive is in the range of about 1 to 5% by weight related to the weight of the fluid-absorbent core.

The basis weight of the fluid-absorbent core is in the range of 600 to 1200 gsm. The density of the fluid-absorbent core is in the range of 0.1 to 0.25 g/cm3. The thickness of the fluid-absorbent core is in the case of diapers in the range of 1 to 5 mm, preferably 1.5 to 3 mm, in the case of incontinence products in the range of 3 to 15 mm.

3. Optional Dusting Layer

An optional component for inclusion into the absorbent core is a dusting layer adjacent to. The dusting layer is a fibrous layer and may be placed on the top and/or the bottom of the absorbent core. Typically, the dusting layer is underlying the storage layer. This underlying layer is referred to as a dusting layer, since it serves as carrier for deposited fluid-absorbent polymer particles during the manufacturing process of the fluid-absorbent core. If the fluid-absorbent polymer material is in the form of macrostructures, films or flakes, the insertion of a dusting layer is not necessary. In the case of fluid-absorbent polymer particles derived from dropletization polymerization, the particles have a smooth surface with no edges. Also in this case, the addition of a dusting layer to the fluid-absorbent core is not necessary. On the other side, as a great advantage the dusting layer provides some additional fluid-handling properties such as wicking performance and may offer reduced incidence of pin-holing and or pock marking of the liquid impervious layer (B).

Preferably, the dusting layer is a fibrous layer comprising fluff (cellulose fibers).

Optional Acquisition-distribution Layer (D)

An optional acquisition-distribution layer (D) is located between the upper layer (A) and the fluid-absorbent core (C) and is preferably constructed to efficiently acquire discharged body fluids and to transfer and distribute them to other regions of the fluid-absorbent composition or to other layers, where the body fluids are immobilized and stored. Thus, the upper layer transfers the discharged liquid to the acquisition-distribution layer (D) for distributing it to the fluid-absorbent core.

The acquisition-distribution layer comprises fibrous material and optionally fluid-absorbent polymer particles.

The fibrous material may be hydrophilic, hydrophobic or can be a combination of both hydrophilic and hydrophobic fibers. It may be derived from natural fibers, synthetic fibers or a combination of both.

Suitable acquisition-distribution layers are formed from cellulosic fibers and/or modified cellulosic fibers and/or synthetics or combinations thereof. Thus, suitable acquisition-distribution layers may contain cellulosic fibers, in particular wood pulp fluff. Examples of further suitable hydrophilic, hydrophobic fibers, as well as modified or unmodified natural fibers are given in the chapter "Liquid-pervious Layer (A)" above.

Especially for providing both fluid acquisition and distribution properties, the use of modified cellulosic fibers is preferred. Examples for modified cellulosic fibers are chemically treated cellulosic fibers, especially chemically stiffened cellulosic fibers. The term "chemically stiffened cellulosic fibers" means cellulosic fibers that have been stiffened by chemical means to increase the stiffness of the fibers. Such means include the addition of chemical stiffening agent in the form of coatings and impregnates. Suitable polymeric stiffening agents can include: cationic modified starches having nitrogen-containing groups, latexes, wet strength resins such as polyamide-epichlorohydrin resin, polyacrylamide, urea formaldehyde and melamine formaldehyde resins and polyethylenimine resins.

Stiffening may also include altering the chemical structure, e.g. by crosslinking polymer chains. Thus crosslinking agents can be applied to the fibers that are caused to chemically form intrafiber crosslink bonds. Further cellulosic fibers may be stiffened by crosslink bonds in individualized form. Suitable chemical stiffening agents are typically monomeric crosslinking agents including C2-C8 dialdehyde, C2-C8 monoaldehyde having an acid functionality, and especially C2-C9 polycarboxylic acids.

Preferably the modified cellulosic fibers are chemically treated cellulosic fibers. Especially preferred are curly fibers which can be obtained by treating cellulosic fibers with citric acid. Preferably the basis weight of cellulosic fibers and modified cellulosic fibers is from 50 to 200 gsm.

Suitable acquisition-distribution layers further include synthetic fibers. Known examples of synthetic fibers are found in the Chapter "Liquid-pervious Layer (A)" above. 3D-poly-ethylene in the function of acquisition-distribution layer is preferred.

Further, as in the case of cellulosic fibers, hydrophilic synthetic fibers are preferred. Hydrophilic synthetic fibers may be obtained by chemical modification of hydrophobic fibers. Preferably, hydrophilization is carried out by surfactant treatment of hydrophobic fibers. Thus the surface of the hydrophobic fiber can be rendered hydrophilic by treatment with a nonionic or ionic surfactant, e.g., by spraying the fiber with a surfactant or by dipping the fiber into a surfactant. Further preferred are permanent hydrophilic synthetic fibers.

The fibrous material of the acquisition-distribution layer may be fixed to increase the strength and the integrity of the layer. Technologies for consolidating fibers in a web are mechanical bonding, thermal bonding and chemical bonding. Detailed description of the different methods of increasing the integrity of the web is given in the Chapter "Liquid-pervious Layer (A)" above.

Preferred acquisition-distribution layers comprise fibrous material and fluid-absorbent polymer particles distributed within. The fluid-absorbent polymer particles may be added during the process of forming the layer from loose fibers, or, alternatively, it is possible to add monomer solution after the formation of the layer and polymerize the coating solution by means of UV-induced polymerization technologies. Thus, "in situ"-polymerization is a further method for the application of fluid-absorbent polymers.

Thus, suitable acquisition-distribution layers comprising from 80 to 100% by weight fibrous material and from 0 to 20% by weight fluid-absorbent polymer particles; preferably from 85 to 99.9% by weight fibrous material and from 0.1 to 15% by weight fluid-absorbent polymer particles; more preferably from 90 to 99.5% by weight fibrous material and from 0.5 to 10% by weight fluid-absorbent polymer particles; and most preferably from 95 to 99% by weight fibrous material and from 1 to 5% by weight fluid-absorbent polymer particles.

Preferred acquisition-distribution layers show basis weights in the range from 20 to 200 gsm, most preferred in the range from 40 to 50 gsm, depending on the concentration of fluid-absorbent polymer particles.

Optional Tissue Layer (E)

An optional tissue layer is disposed immediately above and/or below (C).

The material of the tissue layer may comprise any known type of substrate, including webs, garments, textiles and films. The tissue layer may comprise natural fibers, such as cellulose, cotton, flax, linen, hemp, wool, silk, fur, hair and naturally occurring mineral fibers. The tissue layer may also comprise synthetic fibers such as rayon and lyocell (derived from cellulose), polysaccharides (starch), polyolefin fibers (polypropylene, polyethylene), polyamides, polyester, butadiene-styrene block copolymers, polyurethane and combinations thereof. Preferably, the tissue layer comprises cellulose fibers.

Other Optional Components (F)

1. Leg Cuff

Typical leg cuffs comprising nonwoven materials which can be formed by direct extrusion processes during which the fibers and the nonwoven materials are formed at the same time, or by laying processes of preformed fibers which can be laid into nonwoven materials at a later point of time. Examples for direct extrusion processes include spunbonding, meltblowing, solvent spinning, electrospinning and combinations thereof. Examples of laying processes include wet-laying and dry-laying (e.g. air-laying, carding) methods. Combinations of the processes above include spunbond-meltblown-spunbond (sms), spunbond-meltblow-meltblown-spunbond (smms), spunbond-carded (sc), spunbond-airlaid (sa), meltblown-airlaid (ma) and combinations thereof. The combinations including direct extrusion can be combined at the same point in time or at a subsequent point in time. In the examples above, one or more individual layers can be produced by each process. Thus, "sms" means a three layer nonwoven material, "smsms" or "ssmms" means a five layer nonwoven material. Usually, small type letters (sms) designate individual layers, whereas capital letters (SMS) designate the compilation of similar adjacent layers.

Further, suitable leg cuffs are provided with elastic strands.

Preferred are leg cuffs from synthetic fibers showing the layer combinations sms, smms or smsms. Preferred are nonwovens with the density of 13 to 17 gsm. Preferably leg cuffs are provided with two elastic strands.

2. Elastics

The elastics are used for securely holding and flexibly closing the fluid-absorbent article around the wearer's body, e.g. the waist and the legs to improve containment and fit. Leg elastics are placed between the outer and inner layers or the fluid-absorbent article, or between the outer cover and the bodyside liner. Suitable elastics comprising sheets, ribbons or strands of thermoplastic polyurethane, elastomeric materials, poly(ether-amide) block copolymers, thermoplastic rubbers, styrene-butadiene copolymers, silicon rubbers, natural rubbers, synthetic rubbers, styrene isoprene copolymers, styrene ethylene butylene copolymers, nylon copolymers, spandex fibers comprising segmented polyurethane and/or ethylene-vinyl acetate copolymer. The elastics may be secured to a substrate after being stretched, or secured to a stretched substrate. Otherwise, the elastics may be secured to a substrate and then elastisized or shrunk, e.g. by the application of heat.

3. Closing System

The closing system includes tape tabs, landing zone, elastomerics, pull ups and the belt system.
At least a part of the first waist region is attached to a part of the second waist region by the closing system to hold the fluid-absorbent article in place and to form leg openings and the waist of the fluid-absorbent article. Preferably the fluid-absorbent article is provided with a re-closable closing system.
The closing system is either re-sealable or permanent, including any material suitable for such a use, e.g. plastics, elastics, films, foams, nonwoven substrates, woven substrates, paper, tissue, laminates, fiber reinforced plastics and the like, or combinations thereof. Preferably the closing system includes flexible materials and works smooth and softly without irritating the wearer's skin.
One part of the closing elements is an adhesive tape, or comprises a pair of laterally extending tabs disposed on the lateral edges of the first waist region. Tape tabs are typically attached to the front body panel and extend laterally from each corner of the first waistband. These tape tabs include an adhesive inwardly facing surface which is typically protected prior to use by a thin, removable cover sheet.
Suitable tape tabs may be formed of thermoplastic polymers such as polyethylene, polyurethane, polystyrene, polycarbonate, polyester, ethylene vinyl acetate, ethylene vinyl alcohol, ethylene vinyl acetate acrylate or ethylene acrylic acid copolymers.
Suitable closing systems comprise further a hook portion of a hook and loop fastener and the target devices comprise the loop portion of a hook and loop fastener.
Suitable mechanical closing systems including a landing zone. Mechanical closing systems may fasten directly into the outer cover. The landing zone may act as an area of the fluid-absorbent article into which it is desirable to engage the tape tabs. The landing zone may include a base material and a plurality of tape tabs. The tape tabs may be embedded in the base material of the landing zone. The base material may include a loop material. The loop material may include a backing material and a layer of a nonwoven spunbond web attacked to the backing material.
Thus suitable landing zones can be made by spunbonding. Spunbonded nonwovens are made from melt-spun fibers formed by extruding molten thermoplastic material. Preferred is bioriented polypropylene (BOPP), or brushed/closed loop in the case of mechanical closing systems.
Further, suitable mechanical closing systems including elastic units serving as a flexible waist band for fluid-absorbents articles, such as pants or pull-ups. The elastic units enabling the fluid-absorbent article to be pulled down by the wearer as e.g. a training pant.
Suitable pants-shaped fluid-absorbent article has front section, rear section, crotch section, side sections for connecting the front and rear sections in lateral direction, hip section, elastic waist region and liquid-tight outer layer. The hip section is arranged around the waist of the user. The disposable pants-shaped fluid-absorbent article (pull-up) has favorable flexibility, stretchability, leak-proof property and fit property, hence imparts excellent comfort to the wearer.
Suitable pull-ups comprising thermoplastic films, sheets and laminates having a low modulus, good tear strength and high elastic recovery.
Suitable closing systems may further comprise elastomerics for the production of elastic areas within the fastening devices of the fluid-absorbent article. Elastomerics provide a conformable fit of the fluid-absorbent article to the wearer at the waist and leg openings, while maintaining adequate performance against leakage.

Suitable elastomerics are elastomeric polymers or elastic adhesive materials showing vapor permeability and liquid barrier properties. Preferred elastomerics are retractable after elongation to a length equivalent to its original length.

Suitable closing systems further comprise a belt system, comprising waist-belt and leg-belts for flexibly securing the fluid-absorbent article on the body of the wearer and to provide an improved fit on the wearer. Suitable waist-belts comprising two elastic belts, a left elastic belt, and a right elastic belt. The left elastic belt is associated with each of the left angular edges. The right elastic belt associated with each of the right angular edges. The left and right side belts are elastically extended when the absorbent garment is laid flat. Each belt is connected to and extends between the front and rear of the fluid-absorbent article to form a waist hole and leg holes.

Preferably the belt system is made of elastomerics, thus providing a conformable fit of the fluid-absorbent article and maintaining adequate performance against leakage.

D. Fluid-absorbent Article Construction

The present invention further relates to the joining of the components and layers, films, sheets, tissues or substrates mentioned above to provide the fluid-absorbent article. At least two, preferably all layers, films, sheets, tissues or substrates are joined.

Suitable fluid-absorbent articles include a single- or multiple fluid-absorbent core-system. Preferably fluid-absorbent articles include a single- or double fluid-absorbent core-system.

The construction of the products chassis and the components contained therein is made and controlled by the discrete application of hotmelt adhesives as known to people skilled in the art. Examples would be e.g. H4245, as well as other specific function adhesives manufactured by Bostik S.A., France.

In order to describe the present invention in detail, embodiments are generated which are described hereinafter.

The fluid-absorbent polymer particles and the fluid-absorbent cores are tested by means of the test methods described below.

Methods

The measurements should, unless stated otherwise, be carried out at an ambient temperature of $23 \pm 2°C$ and a relative atmospheric humidity of $50 \pm 10\%$. The fluid-absorbent polymers are mixed thoroughly before the measurement.

Density of the fluid-absorbing polymer particles

The apparent density, also known as bulk density, of the absorbent polymer material, typically in particle form, can be measured according to the standard EDANA test method WSP 260.2 (05), wherein the test conditions, referred to under Section 6.2 of the standard test method, are to be set as $23 \pm 2°C$ and a humidity of $50 \pm 5\%$.

Saline Flow Conductivity (SFC)

The saline flow conductivity is, as described in EP 0 640 330 A1, determined as the gel layer permeability of a swollen gel layer of fluid-absorbent polymer particles, although the apparatus described on page 19 and in figure 8 in the aforementioned patent application was modified to the effect that the glass frit (40) is no longer used, the plunger (39) consists of the same polymer material as the cylinder (37) and now comprises 21 bores having a diameter of 9.65mm each distributed uniformly over the entire contact surface. The procedure and the evaluation of the measurement remains unchanged from EP 0 640 330 A1. The flow rate is recorded automatically.

The saline flow conductivity (SFC) is calculated as follows:

$$SFC \ [cm^3s/g] = (Fg(t=0) \times L0)/(d \times A \times WP),$$

where $Fg(t = 0)$ is the flow rate of NaCl solution in g/s, which is obtained by means of a linear regression analysis of the Fg(t) data of the flow determinations by extrapolation to t = 0, L0 is the thickness of the gel layer in cm, d is the density of the NaCl solution in $g/cm^3$, A is the surface area of the gel layer in $cm^2$ and WP is the hydrostatic pressure over the gel layer in $dyn/cm^2$.

Free Swell Gel Bed Permeability (GBP)

The method to determine the free swell gel bed permeability is described in US 2005/0256757, paragraphs [0061] to [0075].

Centrifuge Retention Capacity (CRC)

The centrifuge retention capacity of the fluid-absorbent polymer particles is determined by the EDANA recommended test method No. WSP 241.2-05 "Centrifuge Retention Capacity", wherein for higher values of the centrifuge retention capacity larger tea bags have to be used.

Particle Size Distribution

The Particle Size Distribution (PSD) of the fluid-absorbent particles is determined according to the EDANA recommended Standard Test No. WSP 220.3.10 "Determination of the particle size distribution by sieve fractionation"

EXAMPLE

Preparation of SAP-Samples

As fluid-absorbent polymer (SAP) Hysorb® B 7075, commercially available water absorbent polymer particles from BASF SE, Ludwigshafen, Germany [CRC= 30,5 g/g ; AUL= 23,5 g/g]), was used.
The SAP had the following Particle size distribution:

>850 $\mu$m = 0.18 %
600-850 $\mu$m = 24.76 %
300-600 $\mu$m = 56.83 %
100-300 $\mu$m = 17.98 %
<100 $\mu$m = 0.25 %

800g of Hysorb® B7075 was sifted in order to collect the fraction with the particle size 100-300 $\mu$m and the fraction 600-850$\mu$m.
SAP sample A was prepared by mixing 100g of the fraction 100-300$\mu$m with 400g of original Hysorb® B 7075 (SAP).
The SAP sample B was prepared by mixing 100g of the fraction 600-850$\mu$m with 400g of original Hysorb® B 7075. The particle size distribution of the prepared samples are summarized in table 1:

Table 1 : Particle size distribution of the different SAP-samples

| Polymer particle | description | PSD (%) | | | | |
|---|---|---|---|---|---|---|
| | | <100 $\mu$m | 100-300 $\mu$m | 300-600 $\mu$m | 600-850 $\mu$m | >850 $\mu$m |
| SAP | HySorb® B7075 | 0.25 | 17.98 | 56.83 | 24.76 | 0.18 |
| SAP sample A | HySorb® B7075 + 20wt% [fraction 100-300 $\mu$m] | 0.18 | 35.21 | 43.60 | 20.77 | 0.24 |
| SAP sample B | HySorb® B7075 + 20wt% [fraction 600-850 $\mu$m] | 0.16 | 16.20 | 50.60 | 32.79 | 0.25 |

Preparation of absorbent cores:

The laminates or absorbent cores (size 30 x 11 cm) were prepared in the laboratory using a rectangular metal box which was connected to a vacuum cleaner. In order to form pockets, a plastic pattern was placed in the box. The pattern was made of a plastic plate in which open rectangular spaces were cut. The design used comprising 24 pockets (rectangular uniform; each 2,0 x 4,0 cm).
A nonwoven (spunmelt PP hydrophilic nonwoven of 10 g/m$^2$ from DOUNOR - France, unwinding tension 1 bar, winding tension 50 N) coated with hotmelt (Bostik, France, adhesive H4245, applied as combed slot coating 2 mm on / 2 mm off with a quantity of about 10 g/m$^2$, spray temperature 145°C) was placed in the box on top of the plastic pattern. Thanks to the vacuum, the nonwoven was suck into the open spaces of the pattern and pockets were created in the nonwoven. The vacuum also helped to immobilize the nonwoven during the preparation of the laminate.

The SAP samples are kept in a closed container not filled by more than 80% of their capacity and rotated by at least five times before taking a portion out of the container.

Samples of 0.5 g are weighed and each pocket was filled with 0.50 g of Superabsorbent Polymer (total of 12 grams of SAP/laminate).

A second nonwoven (spunbond PP nonwoven of 14.5 g/m$^2$ from DOUNOR - France, drive speed 24 m/min, unwinding tension 0 bar, winding tension 50 N) was coated with hotmelt (Bostik adhesive H4245, applied as a full slot coating of about 11 g/m$^2$, spray temperature 135°C, air pressure 0.75 bar, air temperature 145°C) was placed on top of the previous nonwoven. Thus both nonwoven were glued together and the pockets were closed.

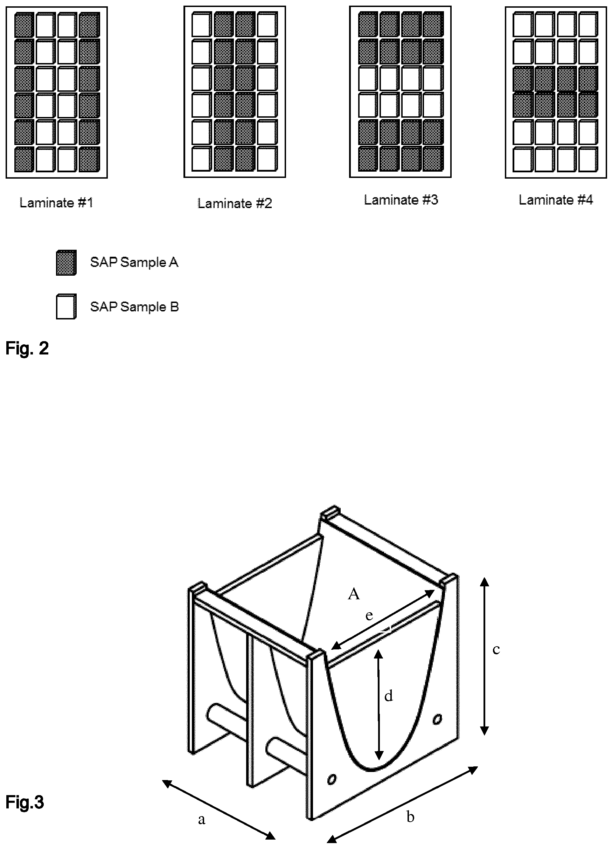

Different cores were prepared with all pockets filled with SAP (laminate #0, inventive core) and cores with pockets filled with SAP sample A or SAP sample B according to specific patterns. Fig. 2 shows the pattern of SAP sample A and SAP sample B on different absorbent cores (laminate #1, laminate #2, laminate #3, laminate #4, all comparative cores).

Absorbent core tested in a diaper

Diapers of the brand dm-Babylove (Aktiv Plus, size 5 Junior, 12-25 kg ; lot 140212-SE071313) were used for testing.

In order to remove the fluff-SAP core, the top-sheet of diapers was cut longitudinally (on the side along the leg-cuffs) and the acquisition layer with top-sheet were flipped aside in order to have access to the absorbing core. The fluff-core was scrapped off. An absorbent core according to the above mentioned preparation method was placed in the position of the fluff-SAP core. The diaper was closed and the top-sheet/acquisition layers were stitched together.

U-shape test

Equipment:

The U-Shape equipment is shown schematically in Fig. 3. It was made of Plexiglas. The supporting box has a dimension of 15.5 x 18.0 x 16.0 cm. The shape was parabolic (d= 14.0 cm and e = 14.0 cm). The parabolic shaped Area A is the contact area/support for the diaper to be measured.

For the measurements the diaper is placed on area A as follows:

First the center of the diaper is determined. Therefore the length of the diaper in both longitudial (LLo) and transverse (LT) direction is measured. The central point (a) is at LLo/2 and LT/2.

The diaper is placed flat with front side upwards into the U-shape equipment, in its longitudinal direction parallel to Area A so that the center of the diaper is almost in the middle of the bottom area of the parabolic shaped support.

Determination of the acquisition time in U-Shape

Scope of the test is the determination of the time that is needed for the diaper to completely absorb a certain amount of synthetic urine to ensure dryness of the diaper even in gush situations. For testing the acquisition rate, the diaper is insulted several times with defined amounts of synthetic urine that is a 0.9% solution of sodium chloride. The acquisition rate is measured by recording the absorption time of a diaper for a certain amount of fluid following multiple separate insults.

According to the method described above diapers were prepared replacing the original absorbent core by each of laminates #0, #1, #2 ,#3, #4.

A diaper (dm-diaper with absorbent core) is then placed in the U-shape equipment as described above. The center of the diaper here the insult point is marked out on the diaper (aquisition point).

70 mL of NaCl solution (0.9 wt% in water) are placed into a funnel. The opening of the funnel positioned centrally on the previously marked insult point. The funnel is opened and the solution poured into the diaper at the previously marked acquisition point. The acquisition time (time for the fluid to be fully absorbed into the core) is recorded in seconds. After 10 minutes have elapsed, the second acquisition can be started.

The procedure as above is repeated for the next insults.

In total 4 acquisitions are measured (each 70 mL of NaCL solution).

The results are summarized in Table 2.

Table 2: Results Acquisition time

| Absorbent core (laminate#) | SAP Sample inner pockets | SAP Sample outer pockets | SAP Sample pockets middle | SAP Samplepockets edges | Acquistion times (seconds) | | | |
|---|---|---|---|---|---|---|---|---|
| | | | | | Time 1 | Time 2 | Time 3 | Time 4 |
| 0 (inventive) | 0 | 0 | | | 68 | 186 | 628 | 900 |
| 1 (comparative) | B | A | --- | --- | 123 | 200 | 420 | >1800 |
| 2 (comparative) | A | B | --- | --- | 91 | 220 | 610 | >1800 |
| 3 (comparative) | --- | --- | B | A | 180 | 290 | 530 | 1150 |
| 4 (comparative) | --- | --- | A | B | 190 | 380 | 820 | 2160 |

The diaper with the inventive core (laminate #0) shows a significant shorter acquisition time than the comparative cores.

**Claims**

1. A fluid-absorbent core comprising

   (A) an upper layer,
   (B) a lower layer,
   (C) fluid-absorbent polymer particles between (A) and (B),

   the upper layer and the lower layer being at least partially joint together by attachments forming a sandwich-like structure with at least some of the unattached regions between the upper layer and the lower layer forming pockets containing fluid-absorbent polymer particles, wherein the particle size distribution (PSD) of the fluid-absorbent polymer particles in one pocket varies from the PSD of the fluid-absorbent polymer particles in any other pocket by not more than 15 %

2. A fluid-absorbent core according to claim 1, wherein the PSD of the fluid-absorbent polymer particles in every pocket varies from the PSD of the fluid-absorbent polymer particles in any other pocket by not more than 10 %, preferably by not more than 5 %, more preferably by not more than 2%.

3. A fluid-absorbent core according to claim 1, wherein the PSD of the fluid-absorbent polymer particles in every pocket is the same.

4. A fluid-absorbent core according to any of claims 1 to 3, wherein the fluid-absorbent core comprises at least 80% by weight of fluid-absorbent polymer particles and less than 20% by weight in total of cellulose and/or synthetic non-cellulose based fibers.

5. A fluid absorbent core according to any of claims 1 to 4, wherein bonding beads are placed between (A) and (B) in the vicinity of the respective pockets.

6. A fluid absorbent core according to any of claims 1 to 5, wherein an adhesive is placed between (A) and (B) in form of at least one layer or stripes or any other form resulting in binding (A) and (B) in areas in the vicinity of the pockets and/or the beads.

7. A fluid-absorbent core according to claim 6, wherein the adhesive is a pressure sensitive adhesive (PSA), preferable a pressure sensitive hotmelt adhesive (HMPSA).

8. A fluid-absorbent core according to any of claims 4 to 7, wherein the synthetic non-cellulose based fibers are based on polyester, polyethylene, polypropylene, polylactic acid, polyamide and/or blends thereof.

9. A fluid-absorbent core according to any of claims 1 to 8, wherein the fluid-absorbent core comprises at least 8 g of fluid-absorbent polymer particles.

10. A fluid-absorbent core according to any of claims 1 to 9, wherein the fluid-absorbent polymer particles have a bulk density of at least 0.55 g/cm$^3$.

11. A fluid-absorbent core according to any of claims 1 to 10, wherein the fluid-absorbent polymer particles have a centrifuge retention capacity of at least 24 g/g.

12. A fluid-absorbent core according to any of claims 1 to 11, wherein the fluid-absorbent polymer particles have absorbency under high load of at least 18 g/g.

13. A fluid-absorbent core according to any of claims 1 to 12, wherein the fluid-absorbent polymer particles have a saline flow conductivity of at least 20x10-7 cm$^3$s/g.

14. A fluid-absorbent article comprising a fluid absorbent core according to any of claims 1 to 13.

15. A fluid-absorbent article according to claim 14, wherein the fluid-absorbent core is encapsulated by wrapping with a nonwoven material or a tissue paper.

**Patentansprüche**

1. Flüssigkeitsabsorbierender Kern, umfassend

   (A) eine obere Schicht,
   (B) eine untere Schicht,
   (C) flüssigkeitsabsorbierende Polymerpartikeln zwischen (A) und (B), wobei die obere Schicht und die untere Schicht wenigstens teilweise über Anhaftungen aneinander gebunden sind und eine sandwichartige Struktur bilden, wobei wenigstens einige der ungebundenen Bereiche zwischen der oberen Schicht und der unteren Schicht Taschen bilden, die flüssigkeitsabsorbierende Polymerpartikeln enthalten, wobei die Partikelgrößenverteilung (PGV) der flüssigkeitsabsorbierenden Polymerpartikeln in einer Tasche zur PGV der flüssigkeitsabsorbierenden Polymerpartikeln in einer anderen Tasche um nicht mehr als 15 % variiert.

2. Flüssigkeitsabsorbierender Kern nach Anspruch 1, wobei die PGV der flüssigkeitsabsorbierenden Polymerpartikeln in jeder Tasche zur PGV der flüssigkeitsabsorbierenden Polymerpartikeln in einer anderen Tasche um nicht mehr als 10 % variiert, vorzugsweise um nicht mehr als 5%, weiter vorzugsweise um nicht mehr als 2 %.

3. Flüssigkeitsabsorbierender Kern nach Anspruch 1, wobei die PGV der flüssigkeitsabsorbierenden Polymerpartikeln in jeder Tasche die Gleiche ist.

4. Flüssigkeitsabsorbierender Kern nach einem der Ansprüche 1 bis 3, wobei der flüssigkeitsabsorbierende Kern wenigstens 80 Gewichts-% an flüssigkeitsabsorbierenden Polymerpartikeln und weniger als 20 Gewichts-% an Zellulose, und/oder synthetischen nicht-Zellulose-basierten Fasern in Summe umfasst.

5. Flüssigkeitsabsorbierender Kern nach einem der Ansprüche 1 bis 4, wobei zwischen (A) und (B) Bondingkügelchen in der Nachbarschaft der entsprechenden Taschen platziert sind.

6. Flüssigkeitsabsorbierender Kern nach einem der Ansprüche 1 bis 5, wobei zwischen (A) und (B) ein Adhäsiv in Form von wenigstens einer Schicht oder Streifen oder einer anderen Form platziert ist, die zur Verbindung von (A) und (B) in Bereichen in der Nachbarschaft der Taschen und/oder der Kügelchen führt.

7. Flüssigkeitsabsorbierender Kern nach Anspruch 6, wobei das Adhäsiv ein druckempfindliches Adhäsiv (PSA, pressure sensitive adhesive), vorzugsweise ein druckempfindliches Hotmelt-Adhäsiv (HMPSA, pressure sensitive hotmelt adhesive) ist.

**8.** Flüssigkeitsabsorbierender Kern nach einem der Ansprüche 4 bis 7, wobei die synthetischen nicht-Zellulose-basierten Fasern auf Polyester, Polyethylen, Polypropylen, Polyessigsäure, Polyamid und/oder Mischungen davon basieren.

**9.** Flüssigkeitsabsorbierender Kern nach einem der Ansprüche 1 bis 8, wobei der flüssigkeitsabsorbierende Kern wenigstens 8 g der flüssigkeitsabsorbierenden Polymerpartikeln umfasst.

**10.** Flüssigkeitsabsorbierender Kern nach einem der Ansprüche 1 bis 9, wobei die flüssigkeitsabsorbierenden Polymerpartikeln eine Schüttdichte von wenigstens 0,55 g/cm$^3$ aufweisen.

**11.** Flüssigkeitsabsorbierender Kern nach einem der Ansprüche 1 bis 10, wobei die flüssigkeitsabsorbierenden Polymerpartikeln eine Zentrifugenrückhaltekapazität von wenigstens 24 g/g aufweisen.

**12.** Flüssigkeitsabsorbierender Kern nach einem der Ansprüche 1 bis 11, wobei die flüssigkeitsabsorbierenden Polymerpartikeln eine Saugfähigkeit unter hoher Belastung von wenigstens 18 g/g aufweisen.

**13.** Flüssigkeitsabsorbierender Kern nach einem der Ansprüche 1 bis 12, wobei die flüssigkeitsabsorbierenden Polymerpartikeln eine Salinefließkonduktivität von wenigstens 20 x 10$^{-7}$ cm$^3$s/g aufweisen.

**14.** Flüssigkeitsabsorbierender Gegenstand, der einen flüssigkeitsabsorbierenden Kern nach einem der Ansprüche 1 bis 13 umfasst.

**15.** Flüssigkeitsabsorbierender Gegenstand nach Anspruch 14, wobei der flüssigkeitsabsorbierende Kern durch Einwickeln mit einem Vliesmaterial oder einem Gewebepapier eingekapselt ist.

## Revendications

**1.** Coeur absorbant des fluides, comprenant :

(A) une couche supérieure,
(B) une couche inférieure,
(C) des particules de polymère absorbant des fluides entre (A) et (B),

la couche supérieure et la couche inférieure étant au moins partiellement jointes ensemble par des liaisons formant une structure de type sandwich, au moins certaines des régions non liées entre la couche supérieure et la couche inférieure formant des poches qui contiennent des particules de polymère absorbant des fluides, dans lequel la distribution des tailles de particules (PSD) des particules de polymère absorbant des fluides dans une poche varie de la PSD des particules de polymère absorbant des fluides dans une autre poche quelconque d'un taux inférieur à 15 %.

**2.** Coeur absorbant des fluides selon la revendication 1, dans lequel la PSD des particules de polymère absorbant des fluides dans chaque poche varie de la PSD des particules de polymère absorbant des fluides dans une autre poche quelconque d'un taux inférieur à 10 %, de préférence d'un taux inférieur à 5 %, plus préférentiellement d'un taux inférieur à 2 %.

**3.** Coeur absorbant des fluides selon la revendication 1, dans lequel la PSD des particules de polymère absorbant des fluides dans chaque poche est la même.

**4.** Coeur absorbant des fluides selon l'une quelconque des revendications 1 à 3, ledit coeur absorbant des fluides comprenant au moins 80 % en poids de particules de polymère absorbant des fluides et moins de 20 % en poids au total de fibres à base de cellulose et/ou de fibres synthétiques non à base de cellulose.

**5.** Coeur absorbant des fluides selon l'une quelconque des revendications 1 à 4, dans lequel les billes de liaison sont placées entre (A) et (B) au voisinage des poches respectives.

**6.** Coeur absorbant des fluides selon l'une quelconque des revendications 1 à 5, dans lequel un adhésif est placé entre (A) et (B) sous la forme d'au moins une couche ou de bandes ou sous toute autre forme quelconque aboutissant à la liaison de (A) et (B) dans des zones situées au voisinage des poches et/ou des billes.

7. Coeur absorbant des fluides selon la revendication 6, dans lequel l'adhésif est un adhésif sensible à la pression (ASP), de préférence un adhésif sensible à la pression thermofondu (ASPT).

8. Coeur absorbant des fluides selon l'une quelconque des revendications 4 à 7, dans lequel les fibres synthétiques non à base de cellulose sont à base de polyester, de polyéthylène, de polypropylène, d'acide polylactique, de polyamide et/ou de mélanges de ceux-ci.

9. Coeur absorbant des fluides selon l'une quelconque des revendications 1 à 8, dans lequel le coeur absorbant des fluides comprend au moins 8 g de particules de polymère absorbant des fluides.

10. Coeur absorbant des fluides selon l'une quelconque des revendications 1 à 9, dans lequel les particules de polymère absorbant des fluides ont une densité apparente d'au moins 0, 55 g/cm$^3$.

11. Coeur absorbant des fluides selon l'une quelconque des revendications 1 à 10, dans lequel les particules de polymère absorbant des fluides ont une capacité de rétention centrifuge d'au moins 24 g/g.

12. Coeur absorbant des fluides selon l'une quelconque des revendications 1 à 11, dans lequel les particules de polymère absorbant des fluides ont une absorbance sous forte charge d'au moins 18 g/g.

13. Coeur absorbant des fluides selon l'une quelconque des revendications 1 à 12, dans lequel les particules de polymère absorbant des fluides ont une conductivité du flux salin d'au moins 20 x 10$^{-7}$ cm$^3$s/g.

14. Article absorbant des fluides comprenant un noyau absorbant des fluides selon l'une quelconque des revendications 1 à 13.

15. Article absorbant des fluides selon la revendication 14, dans lequel le noyau absorbant des fluides est encapsulé par enveloppement avec une matière non tissée ou un papier tissu.

**Fig. 1**

Laminate #1      Laminate #2      Laminate #3      Laminate #4

SAP Sample A

SAP Sample B

**Fig. 2**

**Fig.3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 12199281 A
- US 61740752 B
- EP 1293187 A1
- US 6972011 B
- EP 1447066 A1
- EP 1447067 A1
- EP 1609448 A1
- JP 2004313580 A
- US 20050137085 A
- US 20060004336 A
- US 20070135785 A
- WO 2008155699 A1
- WO 2008155701 A2
- WO 2008155702 A1
- WO 2008155710 A1
- WO 2008155711 A1
- WO 2004071363 A1
- US 20030181115 A
- WO 2005097025 A
- US 2007156108 A
- US 20080125735 A
- WO 2008155722 A2
- WO 2012052172 A
- WO 9837149 A
- US 20050209352 A
- EP 1730218 A
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- WO 2001038402 A1
- DE 3825366 A1
- US 6241928 B
- WO 2008040715 A2
- WO 2008052971 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1
- EP 0640330 A1
- US 20050256757 A
- EP 12199197 A

**Non-patent literature cited in the description**

- **F.L. BUCHHOLZ ; A.T. GRAHAM.** Modern Superabsorbent Polymer Technology. Wiley-VCH, 1998, 252-258
- **ROBERT F. GOULD.** Contact angle, wettability and adhesion. American Chemical Society publication, 1964
- **F. L. BUCHHOLZ ; A. T. GRAHAM.** Modern Superabsorbent Polymer Technology. Wiley-VCH, 1998, 71-103